(19) Europäisches Patentamt
European Patent Office
Office européen des brevets

(11) EP 3 153 172 A1

(12) EUROPEAN PATENT APPLICATION
published in accordance with Art. 153(4) EPC

(43) Date of publication:
**12.04.2017 Bulletin 2017/15**

(21) Application number: **15803771.3**

(22) Date of filing: **04.06.2015**

(51) Int Cl.:
***A61K 31/713*** *(2006.01)* ***A61K 9/127*** *(2006.01)*
***A61K 47/18*** *(2017.01)* ***A61K 48/00*** *(2006.01)*
***A61P 35/00*** *(2006.01)*

(86) International application number:
**PCT/JP2015/066134**

(87) International publication number:
**WO 2015/186770 (10.12.2015 Gazette 2015/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **04.06.2014 JP 2014115814**

(71) Applicants:
- **Kyowa Hakko Kirin Co., Ltd.**
  **Tokyo 100-8185 (JP)**
- **Dicerna Pharmaceuticals, Inc.**
  **Cambridge, MA 02140 (US)**

(72) Inventors:
- **YABUUCHI, Hayato**
  **Tokyo 100-8185 (JP)**
- **KURIHARA, Kana**
  **Tokyo 100-8185 (JP)**
- **YAMADA, Ayumi**
  **Tokyo 100-8185 (JP)**
- **NAKAHASHI, Keiko**
  **Tokyo 100-8185 (JP)**
- **HATANAKA, Kentaro**
  **Tokyo 100-8185 (JP)**
- **TOMURA, Shiho**
  **Tokyo 100-8185 (JP)**

(74) Representative: **Vossius & Partner**
**Patentanwälte Rechtsanwälte mbB**
**Siebertstrasse 3**
**81675 München (DE)**

# (54) CKAP5-GENE-SILENCING RNAI PHARMACEUTICAL COMPOSITION

(57) The present invention provides a composition for suppressing the expression of the CKAP5 gene, the composition comprising a lipid particle containing a double-stranded nucleic acid as a drug and a cationic lipid, the double-stranded nucleic acid having an antisense strand having a base sequence complementary to the sequence of at least 19 continuous bases in CKAP5 gene mRNA of any one of SEQ ID NOs: 1 to 6,
the cationic lipid being represented by formula (I):

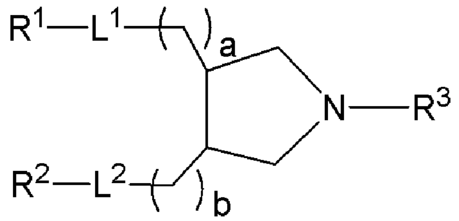

(I)

wherein
$R^1$ and $R^2$ are the same or different and are each linear or branched alkyl, alkenyl or alkynyl of 12 to 24 carbon atoms;
$L^1$ and $L^2$ are the same or different and are each -CO-O- or -O-CO-;
a and b are the same or different and are each 1 to 3; and
$R^3$ is a hydrogen atom, alkyl of 1 to 6 carbon atoms, or alkenyl of 3 to 6 carbon atoms;
a medicament comprising the composition, and the like.

EP 3 153 172 A1

SEQUENCE LISTING

<110>  KYOWA HAKKO KIRIN CO., LTD.
<110>  DICERNA PHARMACEUTICALS, INC.

<120>  RNAi PHARMACEUTICAL COMPOSITION FOR SUPPRESSING EXPRESSION OF CKAP5 GENE

<130>  K04F5192

<150>  JP 2014-115814
<151>  2014-06-04

<160>  20

<170>  PatentIn version 3.5

<210>  1
<211>  21
<212>  RNA
<213>  Homo sapiens

<220>
<223>  Target in CKAP5 mRNA

<400>  1
uuaagugaau uugguuccaa a    21

<210>  2
<211>  21
<212>  RNA
<213>  Homo sapiens

<220>
<223>  Target in CKAP5 mRNA

<400>  2
caacaagaac uagaagcuaa a    21

<210>  3
<211>  21
<212>  RNA
<213>  Homo sapiens

<220>
<223>  Target in CKAP5 mRNA

<400>  3
caguaaugga uaauaaaaau c    21

<210>  4
<211>  21
<212>  RNA
<213>  Homo sapiens

<220>
<223>  Target in CKAP5 mRNA

<400>  4
gauggaaaga aacuaauuuu c    21

<210> 5
<211> 21
<212> RNA
<213> Homo sapiens

<220>
<223> Target in CKAP5 mRNA

<400> 5
ggaugucauu cguaaagaug u 21

<210> 6
<211> 21
<212> RNA
<213> Homo sapiens

<220>
<223> Target in CKAP5 mRNA

<400> 6
acauugaacc auuucugaaa a\201@\201@\201@\201@\201@\201@\201@\201@
21

<210> 7
<211> 25
<212> DNA
<213> Artificial Sequence
<220>
<223> DNA/RNA hybrid molecule

<220>
<223> siRNA sense

<220>
<221> misc_feature
<222> (1)..(23)
<223> RNA

<220>
<221> modified_base
<222> (1)..(2)
<223> um

<220>
<221> modified_base
<222> (4)..(4)
<223> am

<220>
<221> modified_base
<222> (6)..(6)
<223> um

<220>
<221> modified_base
<222> (7)..(7)
<223> gm

<220>

<221> modified_base
<222> (12)..(12)
<223> um

<220>
<221> modified_base
<222> (14)..(14)
<223> gm

<220>
<221> modified_base
<222> (16)..(16)
<223> um

<220>
<221> modified_base
<222> (18)..(18)
<223> cm

<220>
<221> modified_base
<222> (19)..(19)
<223> am

<220>
<221> modified_base
<222> (23)..(23)
<223> um

<400> 7
uuaagugaau uugguuccaa aauca 25

<210> 8
<211> 27
<212> RNA
<213> Artificial Sequence

<220>
<223> siRNA antisense

<220>
<221> modified_base
<222> (1)..(1)
<223> um

<220>
<221> modified_base
<222> (2)..(2)
<223> gm

<220>
<221> modified_base
<222> (3)..(3)
<223> am

<220>
<221> modified_base
<222> (4)..(4)
<223> um

<220>
<221> modified_base

<222> (11)..(11)
<223> am

<220>
<221> modified_base
<222> (13)..(13)
<223> cm

<220>
<221> modified_base
<222> (21)..(21)
<223> cm

<220>
<221> modified_base
<222> (23)..(23)
<223> um

<220>
<221> modified_base
<222> (25)..(25)
<223> am

<220>
<221> modified_base
<222> (26)..(27)
<223> gm

<400> 8
ugauuuugga accaaauuca cuuaagg 27

<210> 9
<211> 25
<212> DNA
<213> Artificial Sequence
<220>
<223> DNA/RNA hybrid molecule

<220>
<223> siRNA sense

<220>
<221> misc_feature
<222> (1)..(23)
<223> RNA

<220>
<221> modified_base
<222> (2)..(2)
<223> am

<220>
<221> modified_base
<222> (4)..(4)
<223> cm

<220>
<221> modified_base
<222> (6)..(6)
<223> am

<220>

<221> modified_base
<222> (8)..(8)
<223> am

<220>
<221> modified_base
<222> (12)..(12)
<223> am

<220>
<221> modified_base
<222> (14)..(14)
<223> am

<220>
<221> modified_base
<222> (16)..(16)
<223> gm

<220>
<221> modified_base
<222> (18)..(18)
<223> um

<400> 9
caacaagaac uagaagcuaa auugg\201@\201@\201@
25

<210> 10
<211> 27
<212> RNA
<213> Artificial Sequence

<220>
<223> siRNA antisense

<220>
<221> modified_base
<222> (1)..(2)
<223> cm

<220>
<221> modified_base
<222> (3)..(4)
<223> am

<220>
<221> modified_base
<222> (11)..(11)
<223> um

<220>
<221> modified_base
<222> (13)..(13)
<223> cm

<220>
<221> modified_base
<222> (21)..(21)
<223> um

<220>

<221> modified_base
<222> (23)..(23)
<223> um

<220>
<221> modified_base
<222> (25)..(27)
<223> gm

<400> 10
ccaauuuagc uucuaguucu uguuggg 27

<210> 11
<211> 25
<212> DNA
<213> Artificial Sequence
<220>
<223> DNA/RNA hybrid molecule

<220>
<223> siRNA sense

<220>
<221> misc_feature
<222> (1)..(23)
<223> RNA

<220>
<221> modified_base
<222> (2)..(2)
<223> am

<220>
<221> modified_base
<222> (5)..(5)
<223> am

<220>
<221> modified_base
<222> (7)..(7)
<223> um

<220>
<221> modified_base
<222> (11)..(11)
<223> um

<220>
<221> modified_base
<222> (12)..(12)
<223> am

<220>
<221> modified_base
<222> (14)..(14)
<223> um

<220>
<221> modified_base
<222> (17)..(18)
<223> am

<400> 11
caguaaugga uaauaaaaau ccaac 25

<210> 12
<211> 27
<212> RNA
<213> Artificial Sequence

<220>
<223> siRNA antisense

<220>
<221> modified_base
<222> (1)..(1)
<223> gm

<220>
<221> modified_base
<222> (2)..(3)
<223> um

<220>
<221> modified_base
<222> (4)..(4)
<223> gm

<220>
<221> modified_base
<222> (11)..(11)
<223> um

<220>
<221> modified_base
<222> (13)..(13)
<223> um

<220>
<221> modified_base
<222> (15)..(15)
<223> am

<220>
<221> modified_base
<222> (17)..(17)
<223> cm

<220>
<221> modified_base
<222> (19)..(19)
<223> am

<220>
<221> modified_base
<222> (21)..(21)
<223> um

<220>
<221> modified_base
<222> (23)..(23)
<223> cm

<220>

<221> modified_base
<222> (25)..(25)
<223> gm

<220>
<221> modified_base
<222> (26)..(26)
<223> cm

<220>
<221> modified_base
<222> (27)..(27)
<223> um

<400> 12
guuggauuuu uauuauccau uacugcu 27

<210> 13
<211> 25
<212> DNA
<213> Artificial Sequence
<220>
<223> DNA/RNA hybrid molecule

<220>
<223> siRNA sense

<220>
<221> misc_feature
<222> (1)..(23)
<223> RNA

<220>
<221> modified_base
<222> (1)..(1)
<223> gm

<220>
<221> modified_base
<222> (2)..(2)
<223> am

<220>
<221> modified_base
<222> (4)..(4)
<223> gm

<220>
<221> modified_base
<222> (6)..(7)
<223> am

<220>
<221> modified_base
<222> (12)..(12)
<223> am

<220>
<221> modified_base
<222> (14)..(14)
<223> um

<220>
<221> modified_base
<222> (16)..(16)
<223> am

<220>
<221> modified_base
<222> (18)..(19)
<223> um

<220>
<221> modified_base
<222> (23)..(23)
<223> gm

<400> 13
gauggaaaga aacuaauuuu caggt 25

<210> 14
<211> 27
<212> RNA
<213> Artificial Sequence

<220>
<223> siRNA antisense

<220>
<221> modified_base
<222> (1)..(1)
<223> am

<220>
<221> modified_base
<222> (2)..(3)
<223> cm

<220>
<221> modified_base
<222> (4)..(4)
<223> um

<220>
<221> modified_base
<222> (11)..(11)
<223> um

<220>
<221> modified_base
<222> (13)..(13)
<223> gm

<220>
<221> modified_base
<222> (15)..(15)
<223> um

<220>
<221> modified_base
<222> (17)..(17)
<223> cm

<220>

<221> modified_base
<222> (19)..(19)
<223> um

<220>
<221> modified_base
<222> (21)..(21)
<223> cm

<220>
<221> modified_base
<222> (23)..(23)
<223> am

<220>
<221> modified_base
<222> (25)..(26)
<223> cm

<220>
<221> modified_base
<222> (27)..(27)
<223> am

<400> 14
accugaaaau uaguuucuuu ccaucca\201@\201@ 27

<210> 15
<211> 25
<212> DNA
<213> Artificial Sequence
<220>
<223> DNA/RNA hybrid molecule

<220>
<223> siRNA sense

<220>
<221> misc_feature
<222> (1)..(23)
<223> RNA

<220>
<221> modified_base
<222> (1)..(1)
<223> gm

<220>
<221> modified_base
<222> (4)..(4)
<223> um

<220>
<221> modified_base
<222> (6)..(6)
<223> um

<220>
<221> modified_base
<222> (12)..(12)
<223> gm

<220>
<221> modified_base
<222> (15)..(15)
<223> am

<220>
<221> modified_base
<222> (18)..(18)
<223> am

<220>
<221> modified_base
<222> (23)..(23)
<223> cm

<400> 15
ggaugucauu cguaaagaug uucgt\201@\201@\201@ 25

<210> 16
<211> 27
<212> RNA
<213> Artificial Sequence

<220>
<223> siRNA antisense

<220>
<221> modified_base
<222> (1)..(1)
<223> am

<220>
<221> modified_base
<222> (2)..(2)
<223> cm

<220>
<221> modified_base
<222> (3)..(3)
<223> gm

<220>
<221> modified_base
<222> (4)..(4)
<223> am

<220>
<221> modified_base
<222> (11)..(11)
<223> um

<220>
<221> modified_base
<222> (13)..(13)
<223> am

<220>
<221> modified_base
<222> (15)..(15)
<223> gm

<220>

<221> modified_base
<222> (17)..(17)
<223> am

<220>
<221> modified_base
<222> (19)..(19)
<223> gm

<220>
<221> modified_base
<222> (21)..(21)
<223> cm

<220>
<221> modified_base
<222> (23)..(23)
<223> um

<220>
<221> modified_base
<222> (25)..(25)
<223> cm

<220>
<221> modified_base
<222> (26)..(27)
<223> um

<400> 16
acgaacaucu uuacgaauga cauccuu\201@\201@\201@
27

<210> 17
<211> 25
<212> DNA
<213> Artificial Sequence
<220>
<223> DNA/RNA hybrid molecule

<220>
<223> siRNA sense

<220>
<221> misc_feature
<222> (1)..(23)
<223> RNA

<220>
<221> modified_base
<222> (2)..(2)
<223> cm

<220>
<221> modified_base
<222> (5)..(5)
<223> um

<220>
<221> modified_base
<222> (7)..(7)
<223> am

<220>
<221> modified_base
<222> (11)..(11)
<223> am

<220>
<221> modified_base
<222> (12)..(12)
<223> um

<220>
<221> modified_base
<222> (14)..(14)
<223> um

<220>
<221> modified_base
<222> (17)..(17)
<223> gm

<220>
<221> modified_base
<222> (18)..(18)
<223> am

<400> 17
acauugaacc auuucugaaa aautc\201@\201@\201@ 25

<210> 18
<211> 27
<212> RNA
<213> Artificial Sequence

<220>
<223> siRNA antisense

<220>
<221> modified_base
<222> (1)..(1)
<223> gm

<220>
<221> modified_base
<222> (2)..(3)
<223> am

<220>
<221> modified_base
<222> (4)..(4)
<223> um

<220>
<221> modified_base
<222> (11)..(11)
<223> gm

<220>
<221> modified_base
<222> (13)..(13)
<223> am

<220>
<221> modified_base
<222> (15)..(15)
<223> um

<220>
<221> modified_base
<222> (17)..(17)
<223> gm

<220>
<221> modified_base
<222> (19)..(19)
<223> um

<220>
<221> modified_base
<222> (21)..(21)
<223> am

<220>
<221> modified_base
<222> (23)..(23)
<223> um

<220>
<221> modified_base
<222> (25)..(25)
<223> um

<220>
<221> modified_base
<222> (26)..(26)
<223> cm

<220>
<221> modified_base
<222> (27)..(27)
<223> am

<400> 18
gaauuuuuca gaaaugguuc aauguca\201@\201@ 27

<210> 19
<211> 25
<212> DNA
<213> Artificial Sequence
<220>
<223> DNA/RNA hybrid molecule

<220>
<223> siRNA sense

<220>
<221> misc_feature
<222> (1)..(23)
<223> RNA

<220>
<221> modified_base
<222> (2)..(2)
<223> am

<220>
<221> modified_base
<222> (4)..(4)
<223> cm

<220>
<221> modified_base
<222> (5)..(6)
<223> am

<220>
<221> modified_base
<222> (8)..(8)
<223> am

<220>
<221> modified_base
<222> (12)..(12)
<223> am

<220>
<221> modified_base
<222> (14)..(14)
<223> am

<220>
<221> modified_base
<222> (16)..(16)
<223> gm

<220>
<221> modified_base
<222> (18)..(18)
<223> um

<400> 19
caacaagaac uagaagcuaa auugg\201@\201@\201@
25

<210> 20
<211> 27
<212> RNA
<213> Artificial Sequence

<220>
<223> siRNA antisense

<220>
<221> modified_base
<222> (1)..(2)
<223> cm

<220>
<221> modified_base
<222> (3)..(4)
<223> am

<220>
<221> modified_base
<222> (9)..(9)
<223> gm

```
<220>
<221>  modified_base
<222>  (11)..(11)
<223>  um

<220>
<221>  modified_base
<222>  (13)..(13)
<223>  cm

<220>
<221>  modified_base
<222>  (15)..(15)
<223>  am

<220>
<221>  modified_base
<222>  (21)..(21)
<223>  um

<220>
<221>  modified_base
<222>  (23)..(23)
<223>  um

<220>
<221>  modified_base
<222>  (25)..(27)
<223>  gm

<400>  20
ccaauuuagc uucuaguucu uguuggg                                          27
```

## Description

TECHNICAL FIELD

The present invention relates to a composition for suppressing the expression of the CKAP5 gene, a medicament comprising the composition, and the like.

BACKGROUND ART

Cytoskeleton-associated protein 5 (CKAP5) is a microtubule-interacting protein that is encoded by the CKAP5 gene in humans (see DNA Res, vol. 2, pp. 37-43, Eur J Biochem, vol. 234, pp. 406-13, Entrez Gene (NCBI (National Center for Biotechnology Information) Gene database): CKAP5 cytoskeleton associated protein 5). CKAP5 is also known as ch-TOG and is the homolog of the *Xenopus* protein XMAP215 (see Molecular Biology of the Cell, vol. 15, pp. 1580-1590).

CKAP5 has at least two distinct roles in microtubule formation: CKAP5 protects kinetochore microtubules from depolymerization by MCAK (KIF2C), and CKAP5 plays a role in centrosome formation (see Molecular and Cellular Biology, vol. 28, pp. 7199-7211). CKAP5 are known to interact with TACC1, which is upregulated in breast cancers (see Oncogene, vol. 22, pp. 8102-16, Biochem J, vol. 363, pp. 195-200, Entrez Gene (NCBI (National Center for Biotechnology Information) Gene database): TACC1 transforming, acidic coiled-coil containing protein 1). CKAP5 are also known to be overexpressed in human liver cancers and large bowel cancers (see Eur J Biochem, vol. 234, pp. 406-13). CKAP5 has been recently identified as a potential target in multiple myeloma, together with MCL1, RRM1, USP8, etc. (see Cancer Res, vol. 72, pp. 757-68). Based on the knowledge, CKAP5 is expected to serve as a good anticancer drug target, but there is yet no effective CKAP5-targeted therapy for cancer etc.

As a method for suppressing the expression of a target gene, for example, a method utilizing RNA interference (hereinafter referred to as RNAi) and the like are known. As previously reported, in particular, introduction of a double-stranded RNA having a sequence identical to that of a target gene into a nematode results in specific suppression of the expression of the target gene (see Nature, vol. 391, No. 6669, pp. 806-811, 1998). It has also been found that, instead of a long double-stranded RNA, introduction of a double-stranded RNA with a length of 21 to 23 bases into a *Drosophila* suppresses the expression of a target gene. Such an RNA is called short interfering RNA (siRNA) (see WO 01/75164).

RNAi has been examined also in many in vivo tests. The effects of siRNAs with a length of 50 base pairs or less on fetal animals (see US 2002/132788) and on adult mice (see WO 03/10180) have been reported. In addition, intravenous administration of siRNAs to a fetal mouse has been found to have the effect of suppressing the expression of a specific gene in various organs such as the kidney, spleen, lung, pancreas and liver (see Nature Genetics, vol. 32, No. 1, pp. 107-108, 2002). It has also been reported that direct administration of siRNAs to the brain results in the suppression of the expression of a specific gene in brain cells (see Nature Biotechnology, vol. 20, No. 10, pp. 1006-1010, 2002).

CKAP5 siRNA is described in, for example, Patent Document 1 etc.

Medicaments containing an siRNA are described in, for example, Patent Documents 2, 3 and 4, etc.

Patent Document 2 discloses medicaments containing an siRNA and, for example, 1,2-dilinoleyloxy-N,N-dimethylaminopropane (DLinDMA), etc. DLinDMA and the like disclosed therein are characterized in that a higher alkyl group of *N*-(2,3-di(9-(*Z*)-octadecenoyloxy)propan-1-yl)-*N,N,N*-trimethy lammonium chloride (DOTAP) and *N*-(2,3-dioleyloxypropan-1-yl)-*N,N,N*-trimethylammonium chloride (DOTMA), which are structurally similar cationic lipids, is converted into a higher alkyl group containing at least two unsaturated bonds in order to develop cationic lipids with higher flexibility and then increase the membrane fluidity of liposome or the like. Patent Document 3 discloses medicaments containing an siRNA and, for example, 2,2-dilinoleyl-4-dimethylaminomethyl-1,3-dioxolane (DLin-K-DMA), etc.

Patent Document 4 discloses, for example, trans-3,4-bis(((Z)-octadeca-9-enoyloxy)methyl)pyrrolidine (Compound I-3) etc.

CITATION LIST

PATENT LITERATURE

Patent Document 1: WO 2004/045543
Patent Document 2: WO 2005/121348
Patent Document 3: WO 2009/086558

Patent Document 4: WO 2011/136368

SUMMARY OF INVENTION

TECHNICAL PROBLEM

An object of the present invention is to provide a composition for suppressing the expression of the CKAP5 gene, a medicament comprising the composition, etc.

SOLUTION TO PROBLEM

The present invention relates to the following (1) to (23).

(1) A composition comprising a lipid particle containing a double-stranded nucleic acid as a drug and a cationic lipid, the double-stranded nucleic acid having a sense strand and an antisense strand, the sense and antisense strands having at least 25 base pairs, the antisense strand having a base sequence complementary to the sequence of at least 19 contiguous bases in CKAP5 gene mRNA of any one of SEQ ID NOs: 1 to 6 and having a maximum length of 35 nucleotides,
the cationic lipid being represented by formula (I):

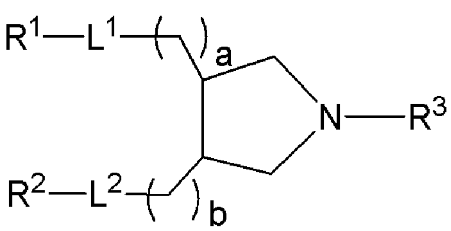

(I)

,

wherein

$R^1$ and $R^2$ are the same or different and are each linear or branched alkyl, alkenyl or alkynyl of 12 to 24 carbon atoms;
$L^1$ and $L^2$ are the same or different and are each -CO-O- or -O-CO-;
a and b are the same or different and are each 1 to 3; and
$R^3$ is a hydrogen atom, alkyl of 1 to 6 carbon atoms, or alkenyl of 3 to 6 carbon atoms.

(2) The composition according to the above (1), wherein the lipid particle further contains a cationic lipid represented by formula (II):

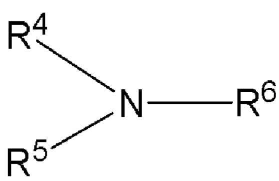

(II)

,

wherein

$R^4$ and $R^5$ are the same or different and are each linear or branched alkyl, alkenyl or alkynyl of 12 to 24 carbon atoms; and
$R^6$ is a hydrogen atom, alkyl of 1 to 6 carbon atoms, alkenyl of 3 to 6 carbon atoms, pyrrolidin-3-yl, piperidin-3-yl, piperidin-4-yl, alkyl of 1 to 6 carbon atoms substituted with a substituent, or alkenyl of 3 to 6 carbon atoms substituted with a substituent, and each substituent is one to three of the same or different groups selected from amino, monoalkylamino, dialkylamino, hydroxy, alkoxy, carbamoyl, monoalkylcarbamoyl, dialkylcarbamoyl, pyrrolidinyl, piperidyl, and morpholinyl.

(3) The composition according to the above (2), wherein $R^4$ and $R^5$ are the same and are dodecyl, tridecyl, tetradecyl, 2,6,10-trimethylundecyl, pentadecyl, 3,7,11-trimethyldodecyl, hexadecyl, heptadecyl, octadecyl, 6,10,14-trimethylpentadecan-2-yl, nonadecyl, 2,6,10,14-tetramethylpentadecyl, icosyl, 3,7,11,15-tetramethylhexadecyl, henicosyl, docosyl, tricosyl, tetracosyl, (*Z*)-tetradec-9-enyl, (*Z*)-hexadec-9-enyl, (*Z*)-octadec-6-enyl, (*Z*)-octadec-9-enyl, (*E*)-octadec-9-enyl, (*Z*)-octadec-11-enyl, (9*Z*,12*Z*)-octadeca-9,12-dienyl, (9*Z*,12*Z*,15*Z*)-octadeca-9,12,15-trienyl, (*Z*)-icos-11-enyl, (11*Z*,14*Z*)-icosa-11,14-dienyl, 3,7,11-trimethyldodeca-2,6,10-trienyl, or 3,7,11,15-tetramethylhexadec-2-enyl.

(4) The composition according to the above (2), wherein $R^4$ and $R^5$ are the same and are tetradecyl, hexadecyl, (*Z*)-hexadec-9-enyl, (*Z*)-octadec-6-enyl, (*Z*)-octadec-9-enyl, (9*Z*,12*Z*)-octadeca-9,12-dienyl, (*Z*)-icos-11-enyl, or (11*Z*,14*Z*)-icosa-11,14-dienyl.

(5) The composition according to the above (3) or (4), wherein $R^6$ is a hydrogen atom, methyl, pyrrolidin-3-yl, piperidin-3-yl, piperidin-4-yl, alkyl of 1 to 6 carbon atoms substituted with a substituent, or alkenyl of 3 to 6 carbon atoms substituted with a substituent, and each substituent is one to three of the same or different groups selected from amino, monoalkylamino, dialkylamino, hydroxy, alkoxy, carbamoyl, monoalkylcarbamoyl, dialkylcarbamoyl, pyrrolidinyl, piperidyl, and morpholinyl.

(6) The composition according to any one of the above (1) to (5), wherein $R^3$ is a hydrogen atom or methyl.

(7) The composition according to any one of the above (1) to (6), wherein $L^1$ and $L^2$ are each -O-CO-; and $R^1$ and $R^2$ are the same and are dodecyl, tetradecyl, hexadecyl, octadecyl, icosyl, docosyl, tetracosyl, (Z)-tetradec-9-enyl, (Z)-hexadec-9-enyl, (Z)-octadec-6-enyl, (Z)-octadec-9-enyl, (E)-octadec-9-enyl, (Z)-octadec-11-enyl, (9Z,12Z)-octadeca-9,12-dienyl, (9Z,12Z,15Z)-octadeca-9,12,15-trienyl, (*Z*)-icos-11-enyl, (11*Z*,14*Z*)-icosa-11,14-dienyl, 3,7,11-trimethyldodeca-2,6,10-trienyl, or 3,7,11,15-tetramethylhexadec-2-enyl.

(8) The composition according to any one of the above (1) to (6), wherein $L^1$ and $L^2$ are each -CO-O-; and $R^1$ and $R^2$ are the same and are tridecyl, pentadecyl, heptadecyl, nonadecyl, henicosyl, tricosyl, (Z)-tridec-8-enyl, (Z)-pentadec-8-enyl, (Z)-heptadec-5-enyl, (Z)-heptadec-8-enyl, (E)-heptadec-8-enyl, (Z)-heptadec-10-enyl, (8Z,11Z)-heptadeca-8,11-dienyl, (8Z,11Z,14Z)-heptadeca-8,11,14-trienyl, (Z)-nonadec-10-enyl, (10Z,13Z)-nonadeca-10,13-dienyl, (11*Z*,14*Z*)-icosa-11,14-dienyl, 2,6,10-trimethylundeca-1,5,9-trienyl, or 2,6,10,14-tetramethylpentadec-1-enyl.

(9) The composition according to any one of the above (1) to (8), wherein the sense and antisense strands of the double-stranded nucleic acid contained as a drug are of SEQ ID NOs: 7 and 8, SEQ ID NOs: 9 and 10, SEQ ID NOs: 11 and 12, SEQ ID NOs: 13 and 14, SEQ ID NOs: 15 and 16, SEQ ID NOs: 17 and 18, or SEQ ID NOs: 19 and 20, respectively.

(10) The composition according to any one of the above (1) to (9), wherein the cationic lipid forms a complex with the double-stranded nucleic acid, or the cationic lipid combined with a neutral lipid and/or a polymer forms a complex with the double-stranded nucleic acid.

(11) The composition according to any one of the above (1) to (9), wherein the cationic lipid forms a complex with the double-stranded nucleic acid, or the cationic lipid combined with a neutral lipid and/or a polymer forms a complex with the double-stranded nucleic acid, and wherein the lipid particle is formed of the complex and a lipid membrane encapsulating the complex.

(12) A method for suppressing the expression of a CKAP5 gene, the method comprising introducing the double-stranded nucleic acid into a cell by using the composition according to any one of the above (1) to (11).

(13) The method according to the above (12), wherein the cell is a cell present in a tumor of a mammal.

(14) The method according to the above (12), wherein the cell is a cell present in the large intestine, pancreas, stomach, small intestine, liver, or esophagus of a mammal.

(15) The method according to any one of the above (12) to (14), wherein the introduction into the cell is achieved by intravenous administration.

(16) A method for treating a CKAP5-associated disease, the method comprising administering the composition according to any one of the above (1) to (11) to a mammal.

(17) The method according to the above (16), wherein the administration is intravenous administration.

(18) A method for treating a cancer, the method comprising administering the composition according to any one of the above (1) to (11) to a mammal.

(19) The method according to the above (18), wherein the administration is intravenous administration.

(20) A medicament comprising the composition according to any one of the above (1) to (11) for use in the treatment of a CKAP5-associated disease.

(21) The medicament for use according to the above (20), wherein the medicament is for intravenous administration.

(22) A therapeutic agent for a cancer, the agent comprising the composition according to any one of the above (1) to (11).

(23) The therapeutic agent for a cancer according to the above (22), which is for intravenous administration.

ADVANTAGEOUS EFFECTS OF INVENTION

Administration of the composition of the present invention to, for example, a mammal suppresses the expression of the CKAP5 gene in the living body, thereby treating a CKAP5-associated disease.

BRIEF DESCRIPTION OF DRAWINGS

Fig. 1 shows the CKAP5 mRNA level in tumors 48 hours after the administration of each of Formulations 1-A and 1-B of Example 1 in an amount equivalent to 10 mg/kg of an siRNA to MIA PaCa-2 xenograft mice. The vertical axis indicates the relative CKAP5 mRNA level. The relative CKAP5 mRNA level was calculated as a semi-quantitative value relative to the CKAP5 mRNA level in a saline administration group, using the mRNA level of a ubiquitously expressed gene, hypoxanthine phosphoribosyltransferase 1 (HPRT1), in each sample as an internal reference.

Fig. 2 shows the CKAP5 mRNA level in tumors 48 hours after the administration of each of Formulations 1-C to 1-F of Example 2 in an amount equivalent to 10 mg/kg of an siRNA to MIA PaCa-2 xenograft mice. The vertical axis indicates the relative CKAP5 mRNA level. The relative CKAP5 mRNA level was calculated as a semi-quantitative value relative to the CKAP5 mRNA level in a saline administration group, using the mRNA level of a ubiquitously expressed gene, hypoxanthine phosphoribosyltransferase 1 (HPRT1), in each sample as an internal reference.

Fig. 3 shows the human CKAP5 mRNA level in tumors 48 hours after the administration of each of Formulations 1-D and 1-E of Example 2 in an amount equivalent to 10 mg/kg of an siRNA to MIA PaCa-2 xenograft mice. The vertical axis indicates the relative CKAP5 mRNA level. The relative CKAP5 mRNA level was calculated as a semi-quantitative value relative to the CKAP5 mRNA level in a saline administration group, using the mRNA level of a ubiquitously expressed gene, hypoxanthine phosphoribosyltransferase 1 (HPRT1), in each sample as an internal reference.

Fig. 4 shows the mouse CKAP5 mRNA level in bone marrow cells 48 hours after the administration of each of Formulations 1-D and 1-E of Example 2 in an amount equivalent to 10 mg/kg of an siRNA to MIA PaCa-2 xenograft mice. The vertical axis indicates the relative CKAP5 mRNA level. The relative CKAP5 mRNA level was calculated as a semi-quantitative value relative to the CKAP5 mRNA level in a saline administration group, using the mRNA level of a ubiquitously expressed gene, hypoxanthine phosphoribosyltransferase 1 (HPRT1), in each sample as an internal reference.

Fig. 5 shows the transition of the relative tumor volume in MIA PaCa-2 xenograft mice to which Formulation 1-B of Example 3 in an amount equivalent to 10 mg/kg of an siRNA was administered on Day 0 and Day 7. The vertical axis indicates the relative tumor volume with the tumor volume on Day 0 taken as 1. The horizontal axis indicates the days elapsed after the start of the experiment. In the chart, the white circles indicate a saline administration group and the black circles indicate a formulation administration group.

Fig. 6 shows the transition of the relative tumor volume in MIA PaCa-2 xenograft mice to which Formulation 1-D of Example 3 in an amount equivalent to 10 mg/kg of an siRNA was administered on Day 0 and Day 7. The vertical axis indicates the relative tumor volume with the tumor volume on Day 0 taken as 1. The horizontal axis indicates the days elapsed after the start of the experiment. In the chart, the white circles indicate a saline administration group and the black circles indicate a formulation administration group.

Fig. 7 shows the transition of the relative tumor volume in MIA PaCa-2 xenograft mice to which Formulation 1-E of Example 3 in an amount equivalent to 10 mg/kg of an siRNA was administered on Day 0 and Day 7. The vertical axis indicates the relative tumor volume with the tumor volume on Day 0 taken as 1. The horizontal axis indicates the days elapsed after the start of the experiment. In the chart, the white circles indicate a saline administration group and the black circles indicate a formulation administration group.

Fig. 8 shows the transition of the relative tumor volume in MIA PaCa-2 xenograft mice to which Formulation 1-G of Example 3 in an amount equivalent to 10 mg/kg of an siRNA was administered on Day 0 and Day 7. The vertical axis indicates the relative tumor volume with the tumor volume on Day 0 taken as 1. The horizontal axis indicates the days elapsed after the start of the experiment. In the chart, the white circles indicate a saline administration group and the black circles indicate a formulation administration group.

Fig. 9 shows the transition of the relative tumor volume in MIA PaCa-2 xenograft mice to which each of Formulations 1-A' and 1-B" of Example 4 in an amount equivalent to 10 mg/kg of an siRNA was administered on Day 0 and Day 7. The vertical axis indicates the relative tumor volume with the tumor volume on Day 0 taken as 1. The horizontal axis indicates the days elapsed after the start of the experiment. In the chart, the white circles indicate a saline administration group, the black circles indicate a formulation 1-A' administration group, and the black triangles indicate a formulation 1-B" administration group.

Fig. 10 shows the transition of the relative tumor volume in MIA PaCa-2 xenograft mice to which each of Formulations

1-B''', 1-C'' and 1-E'' of Example 5 in an amount equivalent to 10 mg/kg of an siRNA was administered on Day 0 and Day 7. The vertical axis indicates the relative tumor volume with the tumor volume on Day 0 taken as 1. The horizontal axis indicates the days elapsed after the start of the experiment. In the chart, the white circles indicate a saline administration group, the black circles indicate a formulation 1-E" administration group, the black triangles indicate a formulation 1-B''' administration group, and the black squares indicate a formulation 1-C'' administration group.

Fig. 11 shows the transition of the relative tumor volume in MIA PaCa-2 xenograft mice to which each of Formulations 1-B''', 1-D'' and 1-F'' of Example 5 in an amount equivalent to 10 mg/kg of an siRNA was administered on Day 0 and Day 7. The vertical axis indicates the relative tumor volume with the tumor volume on Day 0 taken as 1. The horizontal axis indicates the days elapsed after the start of the experiment. In the chart, the white circles indicate a saline administration group, the black circles indicate a formulation 1-D" administration group, the black triangles indicate a formulation 1-B''' administration group, and the black squares indicate a formulation 1-F" administration group.

DESCRIPTION OF EMBODIMENTS

The present invention provides a composition comprising a lipid particle containing a double-stranded nucleic acid as a drug and a cationic lipid, the double-stranded nucleic acid having the ability to reduce or stop the expression of the CKAP5 gene.

The present invention also provides a method for treating a CKAP5-associated disease, the method comprising administering the composition to a mammal to suppress the expression of the CKAP5 gene in the living body.

The present invention also provides a method for treating or preventing a hyperproliferative disorder or disease (for example, leukemia, melanoma, a cell tumor, a cancer, a tumor, an adenoma, etc.) or one or more angiogenic diseases associated with inappropriate expression of the CKAP5 gene.

The lipid particle in the composition of the present invention contains a cationic lipid represented by formula (I) :

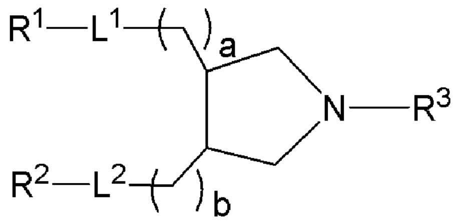

(I)

,

wherein

$R^1$ and $R^2$ are the same or different and are each linear or branched alkyl, alkenyl or alkynyl of 12 to 24 carbon atoms;
$L^1$ and $L^2$ are the same or different and are each -CO-O- or -O-CO-;
a and b are the same or different and are each 1 to 3; and
$R^3$ is a hydrogen atom, alkyl of 1 to 6 carbon atoms, or alkenyl of 3 to 6 carbon atoms.

The compound represented by formula (I) may be hereinafter also referred to as "Compound (I)". The same is also applied to other compounds designated by other numbers.

In another aspect, the lipid particle in the composition of the present invention contains Compound (I) and a cationic lipid represented by formula (II):

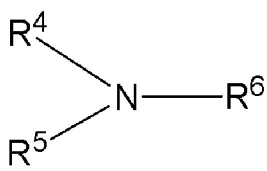

(II)

,

wherein

$R^4$ and $R^5$ are the same or different and are each linear or branched alkyl, alkenyl or alkynyl of 12 to 24 carbon

atoms; and

$R^6$ is a hydrogen atom, alkyl of 1 to 6 carbon atoms, alkenyl of 3 to 6 carbon atoms, pyrrolidin-3-yl, piperidin-3-yl, piperidin-4-yl, alkyl of 1 to 6 carbon atoms substituted with a substituent, or alkenyl of 3 to 6 carbon atoms substituted with a substituent, and each substituent is one to three of the same or different groups selected from amino, monoalkylamino, dialkylamino, hydroxy, alkoxy, carbamoyl, monoalkylcarbamoyl, dialkylcarbamoyl, pyrrolidinyl, piperidyl, and morpholinyl.

In the definition of each group in formula (I), examples of the linear or branched alkyl of 12 to 24 carbon atoms include dodecyl, tridecyl, tetradecyl, 2,6,10-trimethylundecyl, pentadecyl, 3,7,11-trimethyldodecyl, hexadecyl, heptadecyl, octadecyl, 6,10,14-trimethylpentadecan-2-yl, nonadecyl, 2,6,10,14-tetramethylpentadecyl, icosyl, 3,7,11,15-tetramethylhexadecyl, henicosyl, docosyl, tricosyl, and tetracosyl.

In the definition of each group in formula (I), the linear or branched alkenyl of 12 to 24 carbon atoms is linear or branched alkenyl of 12 to 24 carbon atoms having from 1 to 3 double bonds. Examples thereof include (*Z*)-tridec-8-enyl, (*Z*)-tetradec-9-enyl, (*Z*)-pentadec-8-enyl, (*Z*)-hexadec-9-enyl, (*Z*)-heptadec-5-enyl, (*Z*)-octadec-6-enyl, (*Z*)-heptadec-8-enyl, (*Z*)-octadec-9-enyl, (*E*)-heptadec-8-enyl, (*E*)-octadec-9-enyl, (*Z*)-heptadec-10-enyl, (*Z*)-octadec-11-enyl, (8*Z*,11*Z*)-heptadeca-8,11-dienyl, (9*Z*,12*Z*)-octadeca-9,12-dienyl, (8*Z*,11*Z*,14*Z*)-heptadeca-8,11,14-trienyl, (9*Z*,12*Z*,15*Z*)-octadeca-9,12,15-trienyl, (*Z*)-nonadec-10-enyl, (*Z*)-icos-11-enyl, (10*Z*,13*Z*)-nonadeca-10,13-dienyl, (11*Z*,14*Z*)-icosa-11,14-dienyl, 2,6,10-trimethylundeca-1,5,9-trienyl, 3,7,11-trimethyldodeca-2,6,10-trienyl, 2,6,10,14-tetramethylpentadec-1-enyl, and 3,7,11,15-tetramethylhexadec-2-enyl. Of these, preferred are (*Z*)-pentadec-8-enyl, (*Z*)-hexadec-9-enyl, (*Z*)-heptadec-5-enyl, (*Z*)-octadec-6-enyl, (*Z*)-heptadec-8-enyl, (*Z*)-octadec-9-enyl, (8*Z*,11*Z*)-heptadeca-8,11-dienyl, (9*Z*,12*Z*)-octadeca-9,12-dienyl, and the like.

In the definition of each group in formula (I), the linear or branched alkynyl of 12 to 24 carbon atoms is linear or branched alkynyl of 12 to 24 carbon atoms having from 1 to 3 triple bonds. Examples thereof include dodec-11-ynyl, tridec-12-ynyl, pentadec-6-ynyl, hexadec-7-ynyl, pentadeca-4,6-diynyl, hexadeca-5,7-diynyl, heptadec-8-ynyl, and octadec-9-ynyl.

In Compound (I), preferably, $R^1$ and $R^2$ are the same and are linear or branched alkyl, alkenyl or alkynyl of 12 to 24 carbon atoms. $R^1$ and $R^2$ are more preferably linear or branched alkyl or alkenyl of 12 to 24 carbon atoms; and are still more preferably linear alkenyl of 12 to 24 carbon atoms.

In the definition of each group in formula (II), examples of the linear or branched alkyl of 12 to 24 carbon atoms include dodecyl, tridecyl, tetradecyl, 2,6,10-trimethylundecyl, pentadecyl, 3,7,11-trimethyldodecyl, hexadecyl, heptadecyl, octadecyl, 6,10,14-trimethylpentadecan-2-yl, nonadecyl, 2,6,10,14-tetramethylpentadecyl, icosyl, 3,7,11,15-tetramethylhexadecyl, henicosyl, docosyl, tricosyl, and tetracosyl.

In the definition of each group in formula (II), the linear or branched alkenyl of 12 to 24 carbon atoms is linear or branched alkenyl of 12 to 24 carbon atoms having from 1 to 3 double bonds. Examples thereof include (*Z*)-tetradec-9-enyl, (*Z*)-hexadec-9-enyl, (*Z*)-octadec-6-enyl, (*Z*)-octadec-9-enyl, (*E*)-octadec-9-enyl, (*Z*)-octadec-11-enyl, (9*Z*,12*Z*)-octadeca-9,12-dienyl, (9*Z*,12*Z*,15*Z*)-octadeca-9,12,15-trienyl, (*Z*)-icos-11-enyl, (11*Z*,14*Z*)-icosa-11,14-dienyl, 3,7,11-trimethyldodeca-2,6,10-trienyl, and 3,7,11,15-tetramethylhexadec-2-enyl. Of these, preferred are (*Z*)-hexadec-9-enyl, (*Z*)-octadec-6-enyl, (*Z*)-octadec-9-enyl, (9*Z*,12*Z*)-octadeca-9,12-dienyl, (*Z*)-icos-11-enyl, (11*Z*,14*Z*)-icosa-11,14-dienyl, and the like.

In the definition of each group in formula (II), the linear or branched alkynyl of 12 to 24 carbon atoms is linear or branched alkynyl of 12 to 24 carbon atoms having from 1 to 3 triple bonds. Examples thereof include dodec-11-ynyl, tetradec-6-ynyl, hexadec-7-ynyl, hexadeca-5,7-diynyl, and octadec-9-ynyl.

In Compound (II), preferably, $R^4$ and $R^5$ are the same and are linear or branched alkyl, alkenyl or alkynyl of 12 to 24 carbon atoms. $R^4$ and $R^5$ are more preferably linear or branched alkyl or alkenyl of 12 to 24 carbon atoms; and $R^4$ and $R^5$ are still more preferably linear alkenyl of 12 to 24 carbon atoms.

In the definition of each group in formula (I) and formula (II), examples of the alkyl of 1 to 6 carbon atoms include methyl, ethyl, propyl, isopropyl, cyclopropyl, butyl, isobutyl, sec-butyl, tert-butyl, cyclobutyl, cyclopropylmethyl, pentyl, isopentyl, sec-pentyl, neopentyl, tert-pentyl, cyclopentyl, hexyl, and cyclohexyl. Of these, preferred are methyl, ethyl, propyl, isopropyl, butyl, isobutyl, sec-butyl, tert-butyl, pentyl, isopentyl, sec-pentyl, tert-pentyl, neopentyl, hexyl, and the like, and more preferred are methyl, ethyl, propyl, and the like.

Examples of the alkenyl of 3 to 6 carbon atoms include allyl, 1-propenyl, butenyl, pentenyl, and hexenyl. Of these, preferred are allyl and the like.

The alkyl moiety of the alkyl of 1 to 6 carbon atoms substituted with a substituent and the alkenyl moiety of the alkenyl of 3 to 6 carbon atoms substituted with a substituent are as defined for the above alkyl of 1 to 6 carbon atoms and the above alkenyl of 3 to 6 carbon atoms, respectively.

In Compounds (I) and (II), a hydrogen ion may coordinate with the lone pair on the nitrogen atom in the structure. The nitrogen atom whose lone pair coordinates with a hydrogen ion may form a salt together with a pharmaceutically acceptable anion. Each of Compounds (I) and (II) includes a compound in which a hydrogen ion coordinates with the

lone pair on the nitrogen atom.

Examples of the pharmaceutically acceptable anion include inorganic ions such as a chloride ion, a bromide ion, a nitrate ion, a sulfate ion, and a phosphate ion; and organic acid ions such as an acetate ion, an oxalate ion, a maleate ion, a fumarate ion, a citrate ion, a benzoate ion, and a methanesulfonate ion.

In the definition of each group in formula (II), each of pyrrolidin-3-yl, piperidin-3-yl and piperidin-4-yl includes the one in which the hydrogen atom bonded to the nitrogen atom in the ring is converted into methyl or ethyl.

The monoalkylamino or the dialkylamino is amino substituted with one or two of the same or different groups selected from alkyl of 1 to 6 carbon atoms (as defined above) and alkyl of 1 to 6 carbon atoms (as defined above) substituted with amino, methylamino, ethylamino, dimethylamino, diethylamino, pyrrolidinyl, piperidyl, or morpholinyl. Examples thereof include methylamino, ethylamino, propylamino, butylamino, pentylamino, hexylamino, dimethylamino, diethylamino, ethylmethylamino, methylpropylamino, butylmethylamino, methylpentylamino, hexylmethylamino, aminoethylamino, aminopropylamino, (aminoethyl)methylamino, and bis(aminoethyl)amino. Of these, preferred are methylamino, ethylamino, dimethylamino, diethylamino, aminopropylamino, and bis(aminoethyl)amino, and the like.

In Compound (II), the amino, the monoalkylamino and the dialkylamino may form ammonio, monoalkylammonio and dialkylammonio, respectively, through coordination of the lone pair on the nitrogen atom with a hydrogen ion. Thus, the amino, the monoalkylamino, and the dialkylamino include ammonio, monoalkylammonio and dialkylammonio, respectively. In these cases, the ammonio, the monoalkylammonio and the dialkylammonio, resulting from coordination of the lone pair on the nitrogen atom of the amino, the monoalkylamino and the dialkylamino with a hydrogen ion, may form a salt together with a pharmaceutically acceptable anion (as defined above).

The alkoxy is hydroxy substituted with alkyl of 1 to 6 carbon atoms (as defined above) or alkyl of 1 to 6 carbon atoms (as defined above) substituted with amino, methylamino, ethylamino, dimethylamino, diethylamino, pyrrolidinyl, piperidyl, or morpholinyl. Examples thereof include methoxy, ethoxy, propyloxy, butyloxy, pentyloxy, hexyloxy, aminoethoxy, and methylaminoethoxy. Of these, preferred are methoxy, ethoxy, aminoethoxy, methylaminoethoxy, and the like.

The monoalkylcarbamoyl or the dialkylcarbamoyl is carbamoyl substituted with one or two of the same or different groups selected from alkyl of 1 to 6 carbon atoms (as defined above) and alkyl of 1 to 6 carbon atoms (as defined above) substituted with amino, methylamino, ethylamino, dimethylamino, diethylamino, pyrrolidinyl, piperidyl, or morpholinyl. Examples thereof include methylcarbamoyl, ethylcarbamoyl, propylcarbamoyl, butylcarbamoyl, pentylcarbamoyl, hexylcarbamoyl, dimethylcarbamoyl, diethylcarbamoyl, ethylmethylcarbamoyl, methylpropylcarbamoyl, butylmethylcarbamoyl, methylpentylcarbamoyl, hexylmethylcarbamoyl, aminoethylcarbamoyl, aminopropylcarbamoyl, (aminoethyl)methylcarbamoyl, and bis(aminoethyl)carbamoyl. Of these, preferred are methylcarbamoyl, ethylcarbamoyl, dimethylcarbamoyl, and the like.

In Compound (I), when $L^1$ and $L^2$ are each -O-CO-, more preferably, $R^1$ and $R^2$ are the same or different and are each dodecyl, tetradecyl, hexadecyl, octadecyl, icosyl, docosyl, tetracosyl, (*Z*)-tetradec-9-enyl, (*Z*)-hexadec-9-enyl, (*Z*)-octadec-6-enyl, (*Z*)-octadec-9-enyl, (*E*)-octadec-9-enyl, (*Z*)-octadec-11-enyl, (9*Z*,12*Z*)-octadeca-9,12-dienyl, (9*Z*,12*Z*,15*Z*)-octadeca-9,12,15-trienyl, (*Z*)-icos-11-enyl, (11*Z*,14*Z*)-icosa-11,14-dienyl, 3,7,11-trimethyldodeca-2,6,10-trienyl, or 3,7,11,15-tetramethylhexadec-2-enyl; and still more preferably, $R^1$ and $R^2$ are the same or different and are each tetradecyl, hexadecyl, octadecyl, (*Z*)-hexadec-9-enyl, (*Z*)-octadec-6-enyl, (*Z*)-octadec-9-enyl, (9*Z*,12*Z*)-octadeca-9,12-dienyl, (*Z*)-icos-11-enyl, or (11*Z*,14*Z*)-icosa-11,14-dienyl. In either case, more preferably, $R^1$ and $R^2$ are the same.

When $L^1$ and $L^2$ are each -CO-O-, more preferably, $R^1$ and $R^2$ are the same or different and are each tridecyl, pentadecyl, heptadecyl, nonadecyl, henicosyl, tricosyl, (Z)-tridec-8-enyl, (Z)-pentadec-8-enyl, (Z)-heptadec-5-enyl, (Z)-heptadec-8-enyl, (E)-heptadec-8-enyl, (Z)-heptadec-10-enyl, (8Z,11Z)-heptadeca-8,11-dienyl, (8*Z*,11*Z*,14*Z*)-heptadeca-8,11,14-trienyl, (*Z*)-nonadec-10-enyl, (10*Z*,13*Z*)-nonadeca-10,13-dienyl, (11*Z*,14*Z*)-icosa-11,14-dienyl, 2,6,10-trimethylundeca-1,5,9-trienyl, or 2,6,10,14-tetramethylpentadec-1-enyl; and still more preferably, $R^1$ and $R^2$ are the same or different and are each tridecyl, pentadecyl, heptadecyl, (*Z*)-pentadec-8-enyl, (*Z*)-heptadec-5-enyl, (*Z*)-heptadec-8-enyl, (8*Z*,11*Z*)-heptadeca-8,11-dienyl, (*Z*)-nonadec-10-enyl, or (10*Z*,13*Z*)-nonadeca-10,13-dienyl. In either case, preferably, $R^1$ and $R^2$ are the same.

More preferably, a and b are the same and are 1.

In a more preferred embodiment of the present invention, a and b are the same and are 1, and $L^1$ and $L^2$ are each -CO-O-. In this case, more preferably, $R^1$ and $R^2$ are the same or different and are each (*Z*)-heptadec-8-enyl or (8*Z*,11*Z*)-heptadeca-8,11-dienyl, and most preferably, $R^1$ and $R^2$ are the same and are (*Z*)-heptadec-8-enyl or (8*Z*,11*Z*)-heptadeca-8,11-dienyl.

$R^3$ is more preferably a hydrogen atom or methyl.

In a still more preferred embodiment of the present invention, a and b are each 1, $L^1$ and $L^2$ are the same and are -CO-O- or -O-CO-, preferably -CO-O-, and $R^3$ is methyl. More preferably, $R^1$ and $R^2$ are the same or different and are each (*Z*)-heptadec-8-enyl or (8*Z*,11*Z*)-heptadeca-8,11-dienyl, and most preferably, $R^1$ and $R^2$ are the same and are (*Z*)-heptadec-8-enyl or (8*Z*,11*Z*)-heptadeca-8,11-dienyl.

In a more preferred embodiment of the present invention, when $R^3$ is a hydrogen atom, $L^1$ and $L^2$ are the same

and are -CO-O- or -O-CO-, preferably -CO-O-. More preferably, $R^1$ and $R^2$ are the same or different and are each (*Z*)-heptadec-5-enyl or (*Z*)-heptadec-8-enyl, and most preferably $R^1$ and $R^2$ are the same and are (*Z*)-heptadec-5-enyl or (*Z*)-heptadec-8-enyl.

In Compound (II), preferably, $R^4$ and $R^5$ are the same or different and are each tetradecyl, hexadecyl, (*Z*)-tetradec-9-enyl, (*Z*)-hexadec-9-enyl, (*Z*)-octadec-6-enyl, (*Z*)-octadec-9-enyl, (*E*)-octadec-9-enyl, (*Z*)-octadec-11-enyl, (9*Z*,12*Z*)-octadeca-9,12-dienyl, (9*Z*,12*Z*,15*Z*)-octadeca-9,12,15-trienyl, (*Z*)-icos-11-enyl, or (11*Z*,14*Z*)-icosa-11,14-dienyl. More preferably, $R^4$ and $R^5$ are the same or different and are each (*Z*)-octadec-9-enyl or (9*Z*,12*Z*)-octadeca-9, 12-dienyl. Most preferably, $R^4$ and $R^5$ are the same and are (*Z*)-octadec-9-enyl, or (9*Z*,12*Z*)-octadeca-9,12-dienyl.

Preferably, $R^6$ is a hydrogen atom, methyl, pyrrolidin-3-yl, piperidin-3-yl, piperidin-4-yl, alkyl of 1 to 6 carbon atoms substituted with a substituent, or alkenyl of 3 to 6 carbon atoms substituted with a substituent, and each substituent is one to three of the same or different groups selected from amino, monoalkylamino, dialkylamino, hydroxy, alkoxy, carbamoyl, monoalkylcarbamoyl, dialkylcarbamoyl, pyrrolidinyl, piperidyl, and morpholinyl. More preferably, $R^6$ is a hydrogen atom, methyl, alkyl of 1 to 6 carbon atoms substituted with a substituent or alkenyl of 3 to 6 carbon atoms substituted with a substituent, and each substituent is one selected from amino, hydroxy, and carbamoyl. Most preferably, $R^6$ is a hydrogen atom, methyl, or the like.

In a more preferred embodiment of the present invention, $R^6$ is a hydrogen atom. In this case, more preferably, $R^4$ and $R^5$ are the same or different and are each tetradecyl, hexadecyl, (*Z*)-hexadec-9-enyl, (*Z*)-octadec-6-enyl, (*Z*)-octadec-9-enyl, (9*Z*,12*Z*)-octadeca-9,12-dienyl, (*Z*)-icos-11-enyl, or (11*Z*,14*Z*)-icosa-11,14-dienyl, and most preferably, $R^4$ and $R^5$ are the same and are (*Z*)-hexadec-9-enyl, (*Z*)-octadec-9-enyl, or (9*Z*,12*Z*)-octadeca-9,12-dienyl.

In a more preferred embodiment of the present invention, $R^6$ is methyl. In this case, more preferably, $R^4$ and $R^5$ are the same or different and are each tetradecyl, hexadecyl, (*Z*)-hexadec-9-enyl, (*Z*)-octadec-6-enyl, (*Z*)-octadec-9-enyl, (9*Z*,12*Z*)-octadeca-9,12-dienyl, (*Z*)-icos-11-enyl, or (11*Z*,14*Z*)-icosa-11,14-dienyl, and most preferably, $R^4$ and $R^5$ are the same and are (*Z*)-hexadec-9-enyl, (*Z*)-octadec-9-enyl, or (9*Z*,12*Z*)-octadeca-9,12-dienyl.

Compound (I) can be produced in the same manner as in the production method described in WO 2011/136368. In cases where the group(s) defined above change under the conditions of the production method or are inappropriate for carrying out the production method, the introduction and removal of a protective group may be performed in the production of the compound of interest. The introduction and removal of a protective group is commonly adopted in synthetic organic chemistry [for example, see the methods described in Protective Groups in Organic Synthesis, third edition, written by T. W. Greene, John Wiley & Sons Inc. (1999), etc.]. If needed, the order of reaction steps, such as the introduction of a substituent, can be changed.

Next, the production methods of Compound (II) will be described below. In the production methods described below, in cases where the group(s) defined above change under the conditions of the production methods or are inappropriate for carrying out the production methods, the introduction and removal of a protective group may be performed in the production of the compound of interest. The introduction and removal of a protective group is commonly adopted in synthetic organic chemistry [for example, see the methods described in Protective Groups in Organic Synthesis, third edition, written by T. W. Greene, John Wiley & Sons Inc. (1999), etc.]. If needed, the order of reaction steps, such as introduction of a substituent, can be changed.

Production Method 1

Compound (II) can be produced by the following method.

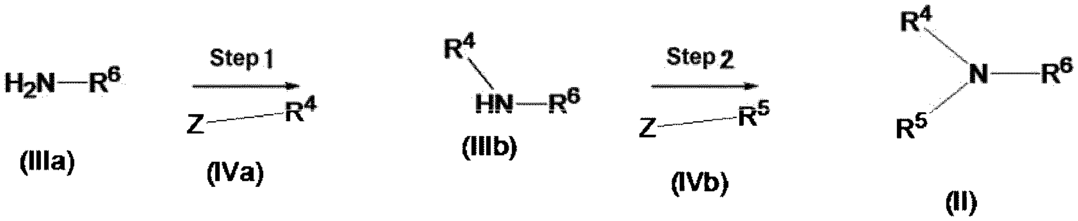


(IIIa) (IVa) (IIIb) (IVb) (II)

In the formulae, $R^4$, $R^5$, and $R^6$ are as defined above, and Z represents a leaving group such as a chlorine atom, a bromine atom, an iodine atom, trifluoromethanesulfonyloxy, methanesulfonyloxy, benzenesulfonyloxy, and p-toluenesulfonyloxy.

Steps 1 and 2

Compound (IIIb) can be produced by reacting Compound (IIIa) and Compound (IVa) in the absence or presence

of a solvent and optionally in the presence of a base in an amount of preferably from 1 to 10 equivalents at a temperature between room temperature and 200°C for from 5 minutes to 100 hours. Compound (II) can be produced by reacting Compound (IIIb) and Compound (IVb) in the absence or presence of a solvent and optionally in the presence of a base in an amount of preferably from 1 to 10 equivalents at a temperature between room temperature and 200°C for from 5 minutes to 100 hours.

Examples of the solvent include methanol, ethanol, dichloromethane, chloroform, 1,2-dichloroethane, toluene, ethyl acetate, acetonitrile, diethyl ether, tetrahydrofuran, 1,2-dimethoxyethane, dioxane, N,N-dimethylformamide, N,N-dimethylacetamide, N-methylpyrrolidone, pyridine, and water. These solvents are used solely or in combination.

Examples of the base include potassium carbonate, potassium hydroxide, sodium hydroxide, sodium methoxide, potassium tert-butoxide, triethylamine, diisopropylethylamine, N-methylmorpholine, pyridine, and 1,8-diazabicyclo[5.4.0]-7-undecene (DBU).

Compound (IIIa) can be obtained as a commercially available product or by a known method (for example, Dai 5-han, Jikken Kagaku Kouza (The Fifth Series of Experimental Chemistry) 14, "Yukikagobutsu no Gosei II", 5th edition, p. 351, Maruzen (2005)) or a method similar thereto.

Each of Compound (IVa) and Compound (IVb) can be obtained as a commercially available product or by a known method (for example, Dai 5-han, Jikken Kagaku Kouza (The Fifth Series of Experimental Chemistry) 13, "Yukikagobutsu no Gosei I", 5th edition, p. 374, Maruzen (2005)) or a method similar thereto.

Compound (IIa), which is Compound (II) wherein $R^4$ and $R^5$ are the same, can be obtained using 2 equivalents or more of Compound (IVa) in Step 1.

Production Method 2

The method described below can be used to produce Compound (IIb), which is Compound (II) wherein $R^6$ is $-CHR^AR^B$ (in the formula, $R^A$ and $R^B$ are the same or different and are each a hydrogen atom, alkyl of 1 to 5 carbon atoms, alkenyl of 2 to 5 carbon atoms, pyrrolidin-2-yl, pyrrolidin-3-yl, piperidin-2-yl, piperidin-3-yl, piperidin-4-yl, morpholin-2-yl, morpholin-3-yl, alkyl of 1 to 5 carbon atoms substituted with a substituent or alkenyl of 2 to 5 carbon atoms substituted with a substituent, and each substituent is one to three of the same or different groups selected from amino, monoalkylamino, dialkylamino, hydroxy, alkoxy, carbamoyl, monoalkylcarbamoyl, dialkylcarbamoyl, pyrrolidinyl, piperidyl, and morpholinyl, or $R^A$ and $R^B$ are taken together with the adjacent carbon atom to form pyrrolidin-3-yl, piperidin-3-yl, or piperidin-4-yl; in cases where $R^A$ and/or $R^B$ is the alkyl, the substituted alkyl, the alkenyl, and/or the substituted alkenyl, the total of the carbon atoms of the alkyl, the alkyl moiety of the substituted alkyl, the alkenyl, and/or the alkenyl moiety of the substituted alkenyl is from 1 to 5, with the exception of the case where $R^A$ and $R^B$ are the same and are a hydrogen atom; in cases where either $R^A$ or $R^B$ is pyrrolidin-2-yl, pyrrolidin-3-yl, piperidin-2-yl, piperidin-3-yl, piperidin-4-yl, morpholin-2-yl, or morpholin-3-yl, the other is a hydrogen atom, alkyl of 1 to 5 carbon atoms, alkenyl of 2 to 5 carbon atoms, pyrrolidin-2-yl, pyrrolidin-3-yl, piperidin-2-yl, piperidin-3-yl, piperidin-4-yl, morpholin-2-yl, morpholin-3-yl, alkyl of 1 to 5 carbon atoms substituted with a substituent or alkenyl of 2 to 5 carbon atoms substituted with a substituent, and each substituent is one or two of the same or different groups selected from amino, monoalkylamino, dialkylamino, hydroxy, alkoxy, carbamoyl, monoalkylcarbamoyl, dialkylcarbamoyl, pyrrolidinyl, piperidyl, and morpholinyl; and in cases where $R^A$ and $R^B$ are each the substituted alkyl or the substituted alkenyl, the total number of the substituents is 2 or 3).

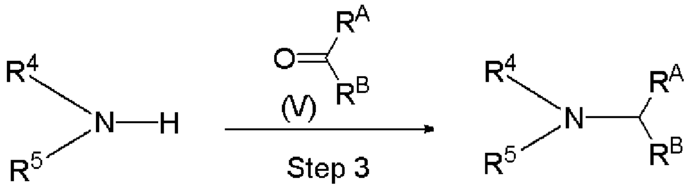

(IIc) (IIb)

In the formulae, $R^4$, $R^5$, $R^A$, and $R^B$ are as defined above.

Step 3

Compound (IIb) can be produced by allowing Compound (IIc), which is Compound (II) wherein $R^6$ is a hydrogen atom, to react with Compound (V) in an amount of preferably from 1 to 10 equivalents in a solvent in the presence of a reducing agent in an amount of preferably from 1 equivalent to an excessively large amount and optionally in the presence of an acid in an amount of preferably from 1 to 10 equivalents at a temperature between -20°C and 150°C for from 5 minutes to 72 hours.

Examples of the solvent include methanol, ethanol, dichloromethane, chloroform, 1,2-dichloroethane, toluene, ethyl acetate, acetonitrile, diethyl ether, tetrahydrofuran, 1,2-dimethoxyethane, dioxane, N,N-dimethylformamide, N,N-dimethylacetamide, N-methylpyrrolidone, and water. These solvents are used solely or in combination.
Examples of the reducing agent include sodium triacetoxyborohydride and sodium cyanoborohydride.
Examples of the acid include hydrochloric acid and acetic acid.
Compound (V) can be obtained as a commercially available product or by a known method (for example, Dai 5-han, Jikken Kagaku Kouza (The Fifth Series of Experimental Chemistry) 15, "Yukikagobutsu no Gosei III", 5th edition, p. 1, Maruzen (2005) ; or Dai 5-han, Jikken Kagaku Kouza (The Fifth Series of Experimental Chemistry) 15, "Yukikagobutsu no Gosei III", 5th edition, p. 153, Maruzen (2005)) or a method similar thereto.

Production Method 3

The method described below can be used to produce Compound (IId), which is Compound (II) wherein $R^6$ is $-CH_2-C(OH)R^CR^D$ (in the formula, $R^C$ and $R^D$ are the same or different and are each a hydrogen atom, alkyl of 1 to 4 carbon atoms, alkenyl of 2 to 4 carbon atoms, pyrrolidin-2-yl, pyrrolidin-3-yl, piperidin-2-yl, piperidin-3-yl, piperidin-4-yl, morpholin-2-yl, morpholin-3-yl, alkyl of 1 to 4 carbon atoms substituted with a substituent or alkenyl of 2 to 4 carbon atoms substituted with a substituent, and each substituent is one or two of the same or different groups selected from amino, monoalkylamino, dialkylamino, hydroxy, alkoxy, carbamoyl, monoalkylcarbamoyl, dialkylcarbamoyl, pyrrolidinyl, piperidyl, and morpholinyl; in cases where $R^C$ and/or $R^D$ is the alkyl, the substituted alkyl, the alkenyl, and/or the substituted alkenyl, the total of the carbon atoms of the alkyl, the alkyl moiety of the substituted alkyl, the alkenyl, and/or the alkenyl moiety of the substituted alkenyl is from 1 to 4, with the exception of the case where $R^C$ and $R^D$ are the same and are a hydrogen atom; in cases where either $R^C$ or $R^D$ is pyrrolidin-2-yl, pyrrolidin-3-yl, piperidin-2-yl, piperidin-3-yl, piperidin-4-yl, morpholin-2-yl, or morpholin-3-yl, the other is a hydrogen atom, alkyl of 1 to 4 carbon atoms, alkenyl of 2 to 4 carbon atoms, pyrrolidin-2-yl, pyrrolidin-3-yl, piperidin-2-yl, piperidin-3-yl, piperidin-4-yl, morpholin-2-yl, morpholin-3-yl, alkyl of 1 to 4 carbon atoms substituted with a substituent or alkenyl of 2 to 4 carbon atoms substituted with a substituent, and each substituent is one selected from amino, monoalkylamino, dialkylamino, hydroxy, alkoxy, carbamoyl, monoalkylcarbamoyl, dialkylcarbamoyl, pyrrolidinyl, piperidyl, and morpholinyl; and in cases where $R^C$ and $R^D$ are each the substituted alkyl or the substituted alkenyl, the total number of the substituents is 2).

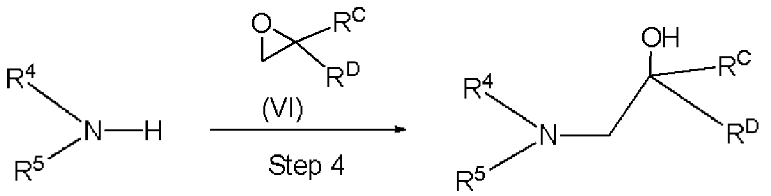

(IIc) (IId)

In the formulae, $R^4$, $R^5$, $R^C$, and $R^D$ are as defined above.

Step 4

Compound (IId) can be produced by reacting Compound (IIc) and Compound (VI) in the absence or presence of a solvent at a temperature between 0°C and 230°C for from 5 minutes to 100 hours.
Examples of the solvent include methanol, ethanol, 1-propanol, dichloromethane, chloroform, 1,2-dichloroethane, toluene, ethyl acetate, acetonitrile, diethyl ether, tetrahydrofuran, 1,2-dimethoxyethane, dioxane, N,N-dimethylformamide, N,N-dimethylacetamide, N-methylpyrrolidone, and dimethyl sulfoxide. These solvents are used solely or in combination.
Compound (VI) can be obtained as a commercially available product or by a known method (for example, Dai 5-han, Jikken Kagaku Kouza (The Fifth Series of Experimental Chemistry) 17, "Yukikagobutsu no Gosei V", 5th edition, p. 186, Maruzen (2005)) or a method similar thereto.
Conversion of the functional groups contained in $R^4$, $R^5$, and $R^6$ in Compound (II) can be carried out by a known method [for example, the method described in Comprehensive Organic Transformations 2nd edition, written by R. C. Larock, Vch Verlagsgesellschaft Mbh (1999), etc.] or a method similar thereto.
The intermediate and the compound of interest in each of the above production methods can be isolated and purified by means of a separation and purification method that is commonly used in synthetic organic chemistry, for example, filtration, extraction, washing, drying, concentration, recrystallization, a variety of chromatographic techniques, etc. Alternatively, the intermediate can be subjected to the subsequent reaction without being purified.

Compounds (I) and (II) may exist as stereoisomers such as geometric isomers and optical isomers, tautomers, and the like. Compounds (I) and (II) include all the possible isomers and mixtures thereof, including stereoisomers and tautomers.

Some or all of the atoms in Compounds (I) and (II) may be replaced by the corresponding isotope atoms. Compounds (I) and (II) include compounds in which some or all of the atoms are replaced by the corresponding isotope atoms. For example, some or all of the hydrogen atoms in Compounds (I) and (II) may be a hydrogen atom having an atomic weight of 2 (a heavy hydrogen atom).

Compounds (I) and (II) in which some or all of the atoms are replaced by the corresponding isotope atoms can be produced using a commercially available building block in the same manner as in the above production methods. Alternatively, Compounds (I) and (II) in which some or all of the hydrogen atoms are replaced by a heavy hydrogen atom(s) can be synthesized by, for example, a method for deuterating an alcohol, a carboxylic acid, or the like using an iridium complex as a catalyst and using heavy water as a heavy hydrogen source (see J. Am. Chem. Soc., vol. 124, No. 10, 2092 (2002)), or other methods.

Specific examples of Compound (I) are shown in Table 1, and specific examples of Compound (II) are shown in Table 2. However, it should not be construed that Compounds (I) and (II) of the present invention are limited thereto.

Table 1

| Compound No. | Structure |
|---|---|
| I-1 | 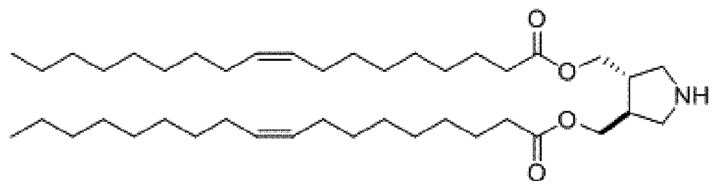 |
| I-2 | 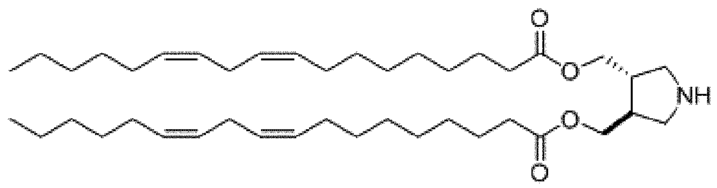 |
| I-3 | 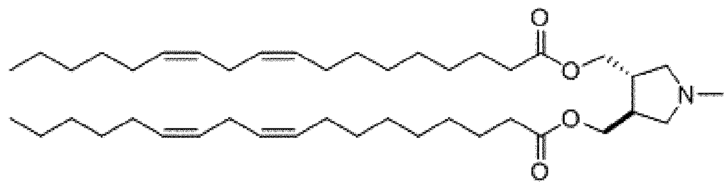 |
| I-4 | 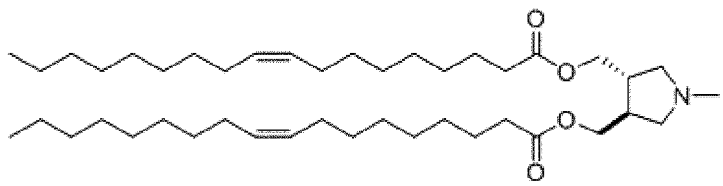 |
| I-5 | 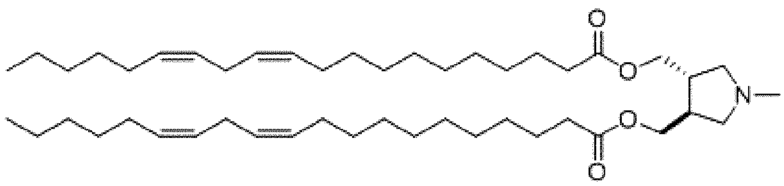 |

Table 2

| Compound No. | Structure |
|---|---|
| II-1 | 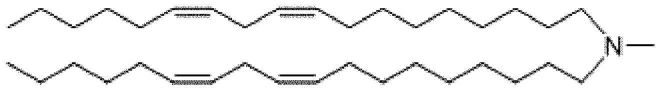 |
| II-2 | 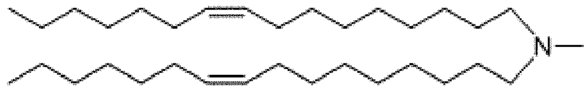 |
| II-3 | 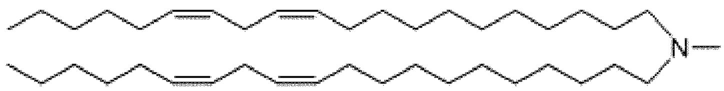 |
| II-4 | 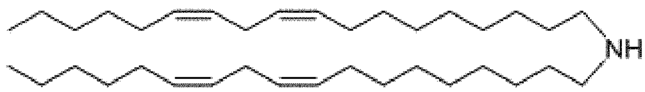 |
| II-5 | 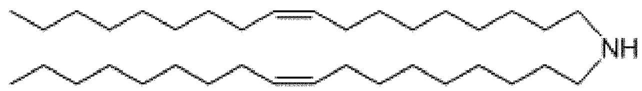 |
| II-6 | 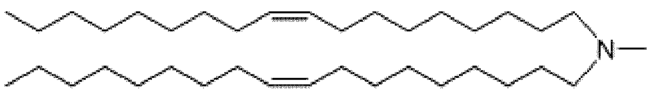 |

The double-stranded nucleic acid used as a drug in the present invention is a double-stranded nucleic acid having the ability to reduce or stop the expression of the CKAP5 gene after introduction into a mammalian cell, the double-stranded nucleic acid having a sense strand and an antisense strand, the sense and antisense strands having at least 25 base pairs, and the antisense strand having a base sequence complementary to the sequence of at least 19 contiguous bases in CKAP5 gene mRNA (CKAP5 mRNA) of any one of SEQ ID NOs: 1 to 6 as a target sequence and having a maximum length of 35 nucleotides.

The double-stranded nucleic acid may be any double-stranded molecule as long as it is a polymer of nucleotides and/or other molecules having an equal function to that of a nucleotide. Examples thereof include RNA, which is a polymer of ribonucleotides; DNA, which is a polymer of deoxyribonucleotides; a chimera nucleic acid composed of RNA and DNA; and a polymer of any of these nucleic acids in which at least one nucleotide is replaced by another molecule having an equal function to that of the nucleotide. The double-stranded nucleic acid of the present invention also includes a derivative containing, as a constituent, at least one molecule obtained through polymerization of nucleotides and/or other molecules having an equal function to that of a nucleotide. The double-stranded nucleic acid of the present invention also includes a peptide nucleic acid (PNA) [Acc. Chem. Res., 32, 624 (1999)], an oxy-peptide nucleic acid (OPNA) [J. Am. Chem. Soc., 123, 4653 (2001)], and a peptide ribonucleic acid (PRNA) [J. Am. Chem. Soc., 122, 6900 (2000)]. In the present invention, uridine (U) in RNA can be read as thymine (T) in DNA, and vice versa.

Examples of said another molecule having an equal function to that of a nucleotide include nucleotide derivatives.

The nucleotide derivatives may be any molecule as long as it is a molecule resulting from modification of a nucleotide. Preferably used is, for example, a molecule resulting from modification of a ribonucleotide or a deoxyribonucleotide for the purpose of enhancing nuclease resistance or stabilization against other decomposing factors as compared with naturally occurring RNA or DNA, increasing the affinity to a complementary nucleic acid strand, increasing penetration through cells, or achieving the visualization.

Examples of the nucleotide derivatives include nucleotides with a modified sugar moiety, nucleotides with a modified phosphodiester bond, and nucleotides with a modified base.

The nucleotides with a modified sugar moiety may be any nucleotide with a modified sugar moiety as long as part or the whole of the chemical structure of the sugar moiety of the nucleotide is modified or substituted with a given substituent, or substituted with a given atom. Preferred is a 2'-modified nucleotide.

Examples of the modifying group in the nucleotide with a modified sugar moiety include 2'-cyano, 2'-alkyl, 2'-substituted alkyl, 2'-alkenyl, 2'-substituted alkenyl, 2'-halogen, 2'-O-cyano, 2'-0-alkyl, 2'-O-substituted alkyl, 2'-O-alkenyl, 2'-O-substituted alkenyl, 2'-S-alkyl, 2'-S-substituted alkyl, 2'-S-alkenyl, 2'-S-substituted alkenyl, 2'-amino, 2'-NH-alkyl, 2'-NH-substituted alkyl, 2'-NH-alkenyl, 2'-NH-substituted alkenyl, 2'-SO-alkyl, 2'-SO-substituted alkyl, 2'-carboxy, 2'-CO-alkyl, 2'-CO-substituted alkyl, 2'-Se-alkyl, 2'-Se-substituted alkyl, 2'-$SiH_2$-alkyl, 2'-$SiH_2$-substituted alkyl, 2'-$ONO_2$, 2'-

$NO_2$, 2'-$N_3$, 2'-amino acid residue (an amino acid residue derived by the removal of the hydroxyl group from the carboxylic acid of an amino acid), and 2'-O-amino acid residue (an amino acid residue as defined above). The 2'-modified nucleotide in the present invention also encompasses bridged nucleic acids (BNAs), in which the 2' -modifying group is bridged to the 4' -carbon atom. Specific examples of BNAs include locked nucleic acids (LNAs), in which the 2'-oxygen atom is bridged to the 4'-carbon atom via methylene, ethylene bridged nucleic acids (ENAs) [Nucleic Acid Research, 32, e175 (2004)], and the like.

Preferred modifying groups in the nucleotide with a modified sugar moiety are 2'-cyano, 2'-halogen, 2'-O-cyano, 2'-alkyl, 2'-substituted alkyl, 2'-0-alkyl, 2'-O-substituted alkyl, 2'-O-alkenyl, 2'-O-substituted alkenyl, 2'-Se-alkyl, 2'-Se-substituted alkyl, and the like. More preferred are 2'-cyano, 2'-fluoro, 2'-chloro, 2'-bromo, 2'-trifluoromethyl, 2'-O-methyl, 2'-O-ethyl, 2'-O-isopropyl, 2'-O-trifluoromethyl, 2'-O-[2-(methoxy)ethyl], 2'-O-(3-aminopropyl), 2'-O-[2-(N,N-dimethyl)aminooxy]ethyl, 2'-O-[3-(N,N-dimethylamino)propyl], 2'-O-{2-[2-(N,N-dimethylamino)ethoxy]ethyl}, 2'-O-[2-(methylamino)-2-oxoethyl], 2'-Se-methyl, and the like. Even more preferred are 2'-0-methyl, 2'-O-ethyl, 2'-fluoro, and the like. Most preferred are 2'-O-methyl and 2'-O-ethyl.

Preferred modifying groups in the nucleotide with a modified sugar moiety can also be defined based on their size. Preferred are modifying groups of a size corresponding to the size of fluoro to the size of -O-butyl, and more preferred are modifying groups of a size corresponding to the size of -O-methyl to the size of -O-ethyl.

The alkyl in the modifying group of the nucleotide with a modified sugar moiety is as defined above for the alkyl of 1 to 6 carbon atoms in Compound (II).

The alkenyl in the modifying group of the nucleotide with a modified sugar moiety is as defined above for the alkenyl of 3 to 6 carbon atoms in Compound (II).

Examples of the halogen in the modifying group of the nucleotide with a modified sugar moiety include a fluorine atom, a chlorine atom, a bromine atom, and an iodine atom.

Examples of the amino acid serving as the source of the above amino acid residue include aliphatic amino acids (specifically, glycine, alanine, valine, leucine, isoleucine, and the like), hydroxy amino acids (specifically, serine, threonine, and the like), acidic amino acids (specifically, aspartic acid, glutamic acid, and the like), acidic amino acid amides (specifically, asparagine, glutamine, and the like), basic amino acids (specifically, lysine, hydroxylysine, arginine, ornithine, and the like), sulfur-containing amino acids (specifically, cysteine, cystine, methionine, and the like), imino acids (specifically, proline, 4-hydroxy proline, and the like), and the like.

Examples of the substituent in the substituted alkyl and the substituted alkenyl in the nucleotide with a modified sugar moiety include halogen (as defined above), hydroxy, sulfanyl, amino, oxo, -O-alkyl (the alkyl moiety of the -O-alkyl is as defined above), -S-alkyl (the alkyl moiety of the -S-alkyl is as defined above), -NH-alkyl (the alkyl moiety of the -NH-alkyl is as defined above), dialkylaminooxy (the two alkyl moieties of the dialkylaminooxy may be the same or different and are each as defined above), dialkylamino (the two alkyl moieties of the dialkylamino may be the same or different and are each as defined above), dialkylaminoalkyleneoxy (the two alkyl moieties of the dialkylaminoalkyleneoxy may be the same or different and are each as defined above, and the alkylene moiety refers to a group resulting from the removal of one hydrogen atom from the above-defined alkyl), and the like. The number of the substituent is preferably 1 to 3.

The nucleotides with a modified phosphodiester bond may be any nucleotides with a modified phosphodiester bond as long as part or the whole of the chemical structure of the phosphodiester bond(s) of the nucleotides are modified or replaced by a given substituent, or substituted with a given atom. Examples of the nucleotides with a modified phosphodiester bond include nucleotides of which the phosphodiester bond (s) are replaced by a phosphorothioate bond, nucleotides of which the phosphodiester bond(s) are replaced by a phosphorodithioate bond, nucleotides of which the phosphodiester bond (s) are replaced by an alkylphosphonate bond, and nucleotides of which the phosphodiester bond(s) are replaced by a phosphoroamidate bond.

The nucleotide with a modified base may be any nucleotide with a modified base as long as part or the whole of the chemical structure of the base of the nucleotide is modified or substituted with a given substituent, or substituted with a given atom. Examples of the nucleotide with a modified base include a nucleotide in which an oxygen atom of the base is replaced by a sulfur atom, a nucleotide in which a hydrogen atom of the base is replaced by an alkyl group of 1 to 6 carbon atoms, a nucleotide in which a methyl group of the base is replaced by a hydrogen atom or an alkyl group of 2 to 6 carbon atoms, and a nucleotide in which an amino group of the base is protected by a protective group such as an alkyl group of 1 to 6 carbon atoms or an alkanoyl group of 1 to 6 carbon atoms.

The nucleotide derivatives in the present invention also include nucleotide derivatives resulting from addition of another chemical substance, such as a lipid, a phospholipid, phenazine, folate, phenanthridine, anthraquinone, acridine, fluorescein, rhodamine, coumarin, and a dye, to a nucleotide or a nucleotide derivative in which at least one of the sugar moiety, the phosphodiester bond, and the base is modified. Specific examples of the nucleotide derivatives with another chemical substance include 5'-polyamine-added nucleotide derivatives, cholesterol-added nucleotide derivatives, steroid-added nucleotide derivatives, bile acid-added nucleotide derivatives, vitamin-added nucleotide derivatives, fluorescence dye Cy5-added nucleotide derivatives, fluorescence dye Cy3-added nucleotide derivatives, 6-fluorescein

(FAM)-added nucleotide derivatives, and biotin-added nucleotide derivatives.

The nucleotide derivatives in the present invention may form a crosslinked structure together with another nucleotide or nucleotide derivative within the double-stranded nucleic acid. The crosslinked structure may be an alkylene structure, a peptide structure, a nucleotide structure, an ether structure, or an ester structure, or a combination of one or more of these structures.

The double-stranded nucleic acid has a sufficient length for processing by Dicer to produce siRNA. In accordance with this embodiment, the double-stranded nucleic acid contains a sense strand preferably having a length between 26 and 30 nucleotides and an antisense strand that anneals to the sense strand under biological conditions, such as the conditions found in the cytoplasm of cells.

The double-stranded nucleic acid may have several properties for enhancing the processing by Dicer. In accordance with this embodiment, the double-stranded nucleic acid has a length sufficient for processing by Dicer to produce siRNA and also has at least one, preferably all, of the following properties (i) to (iii). The first property (i) is that the double-stranded nucleic acid has an asymmetric structure and, for example, has a 3'-overhang in the antisense strand. The second property (ii) is that the sense strand contains a nucleotide derivative (as defined above) at the 3' end for the purposes of Dicer binding and processing. Examples of an appropriate nucleotide derivative include nucleotides such as deoxyribonucleotides, acyclonucleotides, and analogs thereof; and sterically congested molecules such as fluorescent molecules and analogs thereof. Preferred is a deoxyribonucleotide. In cases where such a nucleotide derivative is used, the nucleotide derivative is introduced by replacing a ribonucleotide in the double-stranded nucleic acid in such a manner that the introduction does not result in change in the length of the double-stranded nucleic acid. The third property (iii) is that the sense strand contains a phosphate at the 5'-end. The phrase "contains a phosphate at the 5'-end" means that the hydroxyl group at the 5'-position of the sugar attached to the base at the 5'-end is modified with a phosphate group or another group to be converted into a phosphate group by a nuclease or the like in the living body.

Preferably, the double-stranded nucleic acid has one or more 2' -O-methyl modified nucleotides in the antisense strand or the sense strand or both of the strands. Most preferably, the sense strand contains from 25 to 28 nucleotides, and the two nucleotides from the 3'-end of the sense strand are deoxyribonucleotides. The sense strand contains a phosphate at the 5'-end. The antisense strand contains from 26 to 30 nucleotides and contains a 3'-overhang of from 1 to 4 nucleotides. The antisense strand and the sense strand have one or more 2'-O-methyl modified nucleotides. For example, in cases where the sense strand is a 25-nucleotide sense strand and the antisense strand is a 27-nucleotide antisense strand having a 3'-overhang of two nucleotides and where each of the first bases at the 5'-end of the sense strand and of the antisense strand is designated as position 1, the position of the 2'-O-methyl modification may be, for example, positions 1, 2, 4, 6, 8, 12, 14, 16, 18 and 23 in the sense strand and positions 1, 2, 3, 4, 11, 13, 25 and 27 in the antisense strand; positions 1, 2, 4, 6, 8, 12, 14, 16, 18 and 23 in the sense strand and positions 1, 2, 3, 4, 11, 13, 21, 23, 25, 26 and 27 in the antisense strand; or positions 1, 2, 4, 6, 8, 12, 14, 16, 18 and 23 in the sense strand and positions 1, 2, 3, 4, 11, 13, 15, 17, 19, 21, 23, 25, 26 and 27 in the antisense strand. In all of these cases, preferably the two nucleotides from the 3'-end of the sense strand are deoxyribonucleotides, and the antisense strand contains a phosphate at the 5'-end.

The double-stranded nucleic acid used in the present invention includes derivatives in which an oxygen atom or the like contained in the phosphate moieties, the ester moieties, or the like in the nucleic acid structure is replaced by another atom, for example, a sulfur atom etc.

The 5'-hydroxyl group of the sugar attached to the base at the 5'-end of each of the antisense and sense strands may be modified with a phosphate group, the above modifying group, or another group to be converted into a phosphate group or the above modifying group by a nuclease or the like in the living body.

The 3'-hydroxyl group of the sugar attached to the base at the 3'-end of each of the antisense and sense strands may be modified with a phosphate group, the above modifying group, or another group to be converted into a phosphate group or the above modifying group by a nuclease or the like in the living body.

The double-stranded nucleic acid used in the present invention can be produced by a known RNA or DNA synthetic method or a known RNA or DNA modification method.

The double-stranded nucleic acid can be designed to interact with a target sequence in the CKAP5 gene sequence.

The sequence of either of the strands of the double-stranded nucleic acid is complementary to the target sequence described above. The double-stranded nucleic acid can be chemically synthesized using the methods described herein.

RNA may be produced by enzymatic synthesis or by partial/total organic synthesis. Modified ribonucleotides can be introduced by in vitro enzymatic method or organic synthesis. In one embodiment, each strand is chemically prepared. Synthetic methods of RNA molecules are known in the art [see Nucleic Acids Res., 1988, vol. 32, pp. 936-948]. Generally, the double-stranded nucleic acid can be synthesized using a solid phase oligonucleotide synthetic method (see, for example, Usman et al., US 5, 804, 683; US 5,831,071; US 5,998,203; US 6,117,657; US 6,353,098; US 6,362,323; US 6,437,117; US 6,469,158; Scaringe et al.; US 6,111,086; US 6,008,400; US 6, 111, 086).

The single-stranded nucleic acid is synthesized by solid phase phosphoramidite synthesis (see Nucleic Acids Res., 1993, vol. 30, pp. 2435-2443), then deprotected and desalted on a NAP-5 column (Amersham Pharmacia Biotech, Piscataway, NJ). The oligomer is purified by ion-exchange high performance liquid chromatography (IE-HPLC) on an Amersham Source 15Q column (1.0 cm x height 25 cm, Amersham Pharmacia Biotech, Piscataway, NJ) using a 15 min step-linear gradient. The gradient is run from 90:10 of buffer A:B to 52:48 of buffer A:B, where buffer A is 100 mM Tris, pH 8.5 and buffer B is 100 mM Tris, pH 8.5 (1 M NaCl). The sample is monitored at 260 nm. The fractions with the peak corresponding to the full-length oligonucleotide are collected, pooled, desalted on a NAP-5 column, and lyophilized.

The purity of each single-stranded nucleic acid is determined by capillary electrophoresis (CE) on Beckman PACE 5000 (Beckman Coulter, Inc., Fullerton, Calif). The CE capillary has an inner diameter of 100 μm and contains ssDNA 100-R Gel (Beckman-Coulter). Typically, about 0.6 nmol of the oligonucleotide is injected into the capillary, electrophoresis is run in an electric field of 444 V/cm, and the oligonucleotide is detected by UV absorbance at 260 nm. A denatured Tris-Borate-7 M-urea running buffer is purchased from Beckman-Coulter. The single-stranded nucleic acid with a purity of at least 90% as assessed by CE can be obtained and then used in the experiments described below. Compound identity is examined by matrix-assisted laser desorption ionization time-of-flight (MALDI-TOF) mass spectroscopy on a Voyager DE™ Biospectometry WorkStation (Applied Biosystems, Foster City, Calif.) in accordance with the manufacturer's recommended protocol. The relative molecular mass of the single-stranded nucleic acid obtained can be within ±0.2% of the expected molecular mass.

The single-stranded nucleic acid is resuspended at a concentration of 100 μM in a buffer composed of 100 mM potassium acetate and 30 mM HEPES (pH 7.5). Complementary sense and antisense strands are mixed in equal molar amounts to yield a final 50 μM solution of a double-stranded nucleic acid. The sample is heated to 95°C for 5 minutes and allowed to cool to room temperature before use. The double-stranded nucleic acid is stored at -20°C. The single-stranded nucleic acid is lyophilized or stored in nuclease-free water at -80°C.

Specific examples of the double-stranded nucleic acid used in the present invention are shown in Table 3. In the table, sugars attached to the bases are indicated by r, m and d, which represent a ribose, a ribose whose 2'-hydroxyl group is replaced by -O-methyl, and a deoxyribose, respectively.

Table 3

| Target Sequence of CKAP5 mRNA (5' to 3') | SE Q ID No | Sequence of siRNA antisense (upper row) and sense (lower row) (5' to 3') | SEQ ID No. | siRNA |
|---|---|---|---|---|
| UUAAGUGAAUUUGGUUCCAAA | 1 | mUmUrAmArGmUmGrArArUrUmUrGmGrUmUrCmCmAr ArArAmUdCdA | 7 | A |
| | | mUmGmAmUrUrUrUrGrGrAmArCmCrArArArUrUrCrAmC rUmUrAmAmGmG | 8 | |
| CAACAAGAACUAGAAGCUAAA | 2 | rCmArAmCrAmArGmArArCrUmArGmArAmGrCmUrArAr ArUrUdGdG | 9 | B |
| | | mCmCmAmArUrUrUrArGrCmUrUmCrUrArGrUrUrCrUmU rGmUrUmGmGmG | 10 | |
| CAGUAAUGGAUAAUAAAAAUC | 3 | rCmArGrUmArAmUrGrGrAmUmArAmUrArAmAmArArUr CrCrAdAdC | 11 | C |
| | | mGmUmUmGrGrArUrUrUrUmUrAmUrUmArUmCrCmArU mUrAmCrUmGmCmU | 12 | |
| GAUGGAAAGAAACUAAUUUUC | 4 | mGmArUmGrGmAmArArGrArAmArCmUrAmArUmUmUr UrCrAmGdGdT | 13 | D |
| | | mAmCmCmUrGrArArArArUmUrAmGrUmUrUmCrUmUrU mCrCmArUmCmCmA | 14 | |
| GGAUGUCAUUCGUAAAGAUGU | 5 | mGrGrAmUrGmUrCrArUrUrCmGrUrAmArArGmArUrGrUr UmCdGdT | 15 | E |
| | | mAmCmGmArArCrArUrCrUmUrUmArCmGrAmArUmGrA mCrAmUrCmCmUmU | 16 | |
| ACAUUGAACCAUUUCUGAAAA | 6 | rAmCrArUmUrGmArArCrCmAmUrUmUrCrUmGmArArAr ArArUdTdC | 17 | F |
| | | mGmAmAmUrUrUrUrUrCrAmGrAmArAmUrGmGrUmUrC mArAmUrGmUmCmA | 18 | |
| CAACAAGAACUAGAAGCUAAA | 2 | rCmArAmCmAmArGmArArCrUmArGmArAmGrCmUrArAr ArUrUdGdG | 19 | G |
| | | mCmCmAmArUrUrUrAmGrCmUrUmCrUmArGrUrUrCrU mUrGmUrUmGmGmG | 20 | |

The lipid particle of the present invention comprises the double-stranded nucleic acid with Compound (I) or Compounds (I) and (II). Examples of the lipid particle include a lipid particle containing a complex of the double-stranded nucleic acid with Compound (I) or Compounds (I) and (II); a lipid particle containing a complex of the double-stranded nucleic acid with Compound (I) or Compounds (I) and (II) combined with a neutral lipid and/or a polymer; and a lipid particle formed of such a complex and a lipid membrane encapsulating the complex. The lipid membrane may be either a lipid monolayer membrane (lipid monomolecular membrane) or a lipid bilayer membrane (lipid bimolecular membrane). The lipid membrane may contain Compound (I), Compound (II), a neutral lipid, and/or a polymer. The complex and/or the lipid membrane may contain a cationic lipid other than Compound (I) or (II).

Further examples of the lipid particle include a lipid particle formed of a complex and a lipid membrane, wherein the complex is a complex of the double-stranded nucleic acid with Compound (II) or a complex of the double-stranded nucleic acid with Compound (II) combined with a neutral lipid and/or a polymer and wherein the lipid membrane encapsulates the complex and contains Compound (I). Also in this embodiment, the lipid membrane may be either a lipid monolayer membrane (lipid monomolecular membrane) or a lipid bilayer membrane (lipid bimolecular membrane). The lipid membrane may further contain Compound (II), a neutral lipid and/or a polymer. The complex and/or the lipid membrane may further contain a cationic lipid other than Compound (I) or (II).

The form of the complex in any of the embodiments of the present invention includes, for example, a complex of the double-stranded nucleic acid and a lipid monolayer membrane (reverse micelle), a complex of the double-stranded nucleic acid and a liposome, and a complex of the double-stranded nucleic acid and a micelle. Of these, preferred are a complex of the double-stranded nucleic acid and a lipid monolayer membrane, and a complex of the double-stranded nucleic acid and a liposome.

Examples of the lipid particle formed of the above complex and a lipid bilayer membrane encapsulating the complex include a liposome formed of the complex and a lipid bilayer membrane encapsulating the complex.

The lipid particle of the present invention may contain Compounds (I) and (II) that each may be a single type or two or more types. Compound (I) and/or Compound (II) may be mixed with a cationic lipid other than Compound (I) or (II).

Examples of the cationic lipid other than Compound (I) or (II) include DOTMA, DOTAP, and the like as disclosed in JP S61-161246 A (US 5049386); *N*-[1-(2,3-dioleyloxypropyl)]-*N,N*-dimethyl-*N*-hydroxyethylamm onium bromide (DORIE), 2,3-dioleyloxy-*N*-[2-(sperminecarboxamido)ethyl]-*N,N*-dimethy 1-1-propanaminium trifluoroacetate (DOSPA), and the like as disclosed in WO 91/16024 and WO 97/019675; DLinDMA and the like as disclosed in WO 2005/121348; and DLin-K-DMA and the like as disclosed in WO 2009/086558. The cationic lipid other than Compound (I) or (II) is preferably a cationic lipid with a tertiary amine moiety having two unsubstituted alkyl groups or with a quaternary ammonium moiety having three unsubstituted alkyl groups, such as DOTMA, DOTAP, DORIE, DOSPA, DLinDMA, and DLin-K-DMA, and is more preferably a cationic lipid with such a tertiary amine moiety. Each unsubstituted alkyl group in the tertiary amine moiety and the quaternary ammonium moiety is more preferably a methyl group.

The lipid particle of the present invention can be produced by a known production method or a method similar thereto, and the lipid particle may be one produced by any production method. For example, for the production of a liposome as one embodiment of the lipid particle, a known method for preparing a liposome can be used. Examples of a known method for preparing a liposome include the liposome preparation method by Bangham et al. (see J. Mol. Biol., 1965, vol. 13, pp. 238-252); the ethanol injection method (see J. Cell Biol., 1975, vol. 66, pp. 621-634); the French press method (see FEBS Lett., 1979, vol. 99, pp. 210-214); the freeze-thawing method (see Arch. Biochem. Biophys., 1981, vol. 212, pp. 186-194); the reverse-phase evaporation method (see Proc. Natl. Acad. Sci. USA, 1978, vol. 75, pp. 4194-4198); and the pH gradient method (see, for example, Japanese Patent Nos. 2572554 and 2659136, etc.). A liquid in which a liposome is dispersed in the liposome production process may be, for example, water, an acid, an alkali, a variety of buffer solutions, physiological saline, an amino acid infusion, and the like. In the production of a liposome, an additive can also be added, including an antioxidant such as citric acid, ascorbic acid, cysteine, and ethylenediaminetetraacetic acid (EDTA), and an isotonic agent such as glycerol, glucose, and sodium chloride. A liposome can also be produced by a method including the steps of dissolving a lipid or the like in an organic solvent such as ethanol, evaporating the solvent, adding physiological saline or the like thereto, and stirring and shaking the mixture to form a liposome.

The lipid particle of the present invention can be produced by, for example, a method including the steps of dissolving, in chloroform, Compound (I) alone, a combination of Compounds (I) and (II), a combination of Compound (I) and a cationic lipid other than Compound (I) or (II), or a combination of Compounds (I) and (II) and a cationic lipid other than Compound (I) or (II), then adding, to the solution, an aqueous double-stranded nucleic acid solution and methanol followed by mixing to form a complex of the cationic lipid(s) and the double-stranded nucleic acid, separating the chloroform layer, adding a PEGylated phospholipid, a neutral lipid and water to the layer to form a water-in-oil (W/O) emulsion, and subjecting the emulsion to reverse-phase evaporation (see JP 2002-508765 T); a method including the steps of dissolving the double-stranded nucleic acid in an acidic aqueous electrolyte solution, adding a lipid (in ethanol), reducing the ethanol concentration to 20 v/v% to prepare liposomes encapsulating the double-stranded nucleic acid, subjecting the liposomes to filter sizing, dialyzing the liposomes to remove the excessive ethanol, increasing the pH of the resulting sample followed by further dialysis to remove the double-stranded nucleic acid attached to the surface of the liposomes

(see JP 2002-501511 T and Biochimica et Biophysica Acta, 2001, Vol. 1510, pp. 152-166); or other methods.

As a particular example of the lipid particle of the present invention, the liposome formed of the complex and a lipid bilayer membrane encapsulating the complex can be produced in accordance with the production method described in, for example, WO 02/28367, WO 2006/080118, etc.

Some other particular examples of the lipid particle of the present invention, for example, a lipid particle formed of a complex and a lipid membrane encapsulating the complex, wherein the complex is a complex of the double-stranded nucleic acid with Compound (I) or Compounds (I) and (II) or a complex of the double-stranded nucleic acid with Compound (I) or Compounds (I) and (II) combined with a neutral lipid and/or a polymer, and wherein the lipid membrane contains Compound (I), Compound (II), and/or a cationic lipid other than Compound (I) or (II) ; a lipid particle formed of a complex and a lipid membrane encapsulating the complex, wherein the complex is a complex of the double-stranded nucleic acid with Compound (II) or a complex of the double-stranded nucleic acid with Compound (II) combined with a neutral lipid and/or a polymer, and wherein the lipid membrane contains Compound (I) or Compounds (I) and (II); or the like, can be produced by a method including the steps of preparing each complex in accordance with the production method described in WO 02/28367, WO 2006/080118, etc., dispersing (not dissolving) the complex in water or a 0 to 20% ethanol aqueous solution to prepare a dispersion (liquid A), separately dissolving the components of the lipid membrane in an ethanol aqueous solution to prepare a solution (liquid B), mixing liquids A and B, and adding an appropriate amount of water. Compound (I), Compound (II), and the cationic lipid other than Compound (I) or (II) in the liquids A and B may be a single type or two or more types thereof.

In the present invention, the lipid particle, for example, a lipid particle formed of a complex and a lipid membrane encapsulating the complex, wherein the complex is a complex of the double-stranded nucleic acid with Compound (I) or Compounds (I) and (II) or a complex of the double-stranded nucleic acid with Compound (I) or Compounds (I) and (II) combined with a neutral lipid and/or a polymer, and wherein the lipid membrane contains Compound (I), Compound (II), and/or a cationic lipid other than Compound (I) or (II); a lipid particle formed of a complex and a lipid membrane encapsulating the complex, wherein the complex is a complex of the nucleic acid with Compound (II) or a complex of the nucleic acid with Compound (II) combined with a neutral lipid and/or a polymer, and wherein the lipid membrane contains Compound (I) or Compounds (I) and (II); or the like, may be the one in which the structures of the complex and of the lipid membrane have been changed during and/or after the production process due to the electrostatic interaction between the double-stranded nucleic acid contained in the complex and the cationic lipid contained in the lipid membrane, or due to fusion between the cationic lipid contained in the complex and the cationic lipid contained in the lipid membrane.

A more preferred lipid particle of the present invention is a lipid particle formed of a complex and a lipid membrane encapsulating the complex, wherein the complex is a complex of the double-stranded nucleic acid with Compound (I) or Compounds (I) and (II) or a complex of the double-stranded nucleic acid with Compound (I) or Compounds (I) and (II) combined with a neutral lipid and/or a polymer, and wherein the lipid membrane contains Compound (I), Compound (II), or a cationic lipid other than Compound (I) or (II); more preferred is a lipid particle formed of a complex and a lipid membrane encapsulating the complex, wherein the complex is a complex of the double-stranded nucleic acid with Compound (I) or Compounds (I) and (II) or a complex of the double-stranded nucleic acid with Compound (I) or Compounds (I) and (II) combined with a neutral lipid and/or a polymer, and wherein the lipid membrane contains Compound (I) or Compounds (I) and (II) ; and still more preferred are a lipid particle formed of a complex and a lipid membrane encapsulating the complex, wherein the complex is a complex of the double-stranded nucleic acid with Compound (I) alone or combined with a neutral lipid and wherein the lipid membrane contains Compound (I), and a lipid particle formed of a complex and a lipid membrane encapsulating the complex, wherein the complex is a complex of the double-stranded nucleic acid with Compound (II) alone or combined with a neutral lipid and wherein the lipid membrane contains Compounds (I) and (II).

The total number of the molecules of Compounds (I) and (II) in the complex in the present invention is preferably 0.5 to 4 times the total number of the phosphorus atoms in the double-stranded nucleic acid, and is more preferably 1.5 to 3.5 times, further more preferably 2 to 3 times the total number of the phosphorus atoms. The total number of the molecules of Compounds (I) and (II), and the cationic lipid other than Compound (I) or (II) in the complex of the present invention is preferably 0.5 to 4 times the total number of the phosphorus atoms in the double-stranded nucleic acid, and is more preferably 1.5 to 3. 5 times, further more preferably 2 to 3 times the total number of the phosphorus atoms.

In cases where the lipid particle of the present invention is formed of a complex and a lipid membrane encapsulating the complex, the total number of the molecules of Compounds (I) and (II) in the lipid particle is preferably 1 to 10 times the total number of the phosphorus atoms in the double-stranded nucleic acid, and is more preferably 2.5 to 9 times, further more preferably 3.5 to 8 times the total number of the phosphorus atoms. The total number of the molecules of Compounds (I) and (II), and the cationic lipid other than Compound (I) or (II) in the lipid particle is preferably 1 to 10 times the total number of the phosphorus atoms in the double-stranded nucleic acid, and is more preferably 2.5 to 9 times, further more preferably 3.5 to 8 times the total number of the phosphorus atoms.

The neutral lipid may be any neutral lipid including simple lipids, complex lipids, and derived lipids. Examples of the neutral lipid include phospholipids, glyceroglycolipids, sphingoglycolipids, sphingoids, and sterols, but are not

limited thereto.

In cases where the lipid particle of the present invention contains such a neutral lipid, the total number of the molecules in the neutral lipid is preferably 0.1 to 1.8 times the total number of the molecules of Compounds (I) and (II), and the cationic lipid other than Compound (I) or (II), and is more preferably 0.3 to 1.1 times, further more preferably 0.4 to 0.9 times the total number of the molecules of Compounds (I) and (II), and the cationic lipid other than Compound (I) or (II). The lipid particle of the present invention may contain the neutral lipid in the complex or in the lipid membrane encapsulating the complex. More preferably, the neutral lipid is contained at least in the lipid membrane encapsulating the complex, and still more preferably, the neutral lipid is contained both in the complex and in the lipid membrane encapsulating the complex.

Examples of the phospholipid as the neutral lipid include natural or synthetic phospholipids, such as phosphatidylcholines (specifically, soybean phosphatidylcholine, egg yolk phosphatidylcholine (EPC), distearoylphosphatidylcholine (DSPC), dipalmitoylphosphatidylcholine (DPPC), palmitoyloleoylphosphatidylcholine (POPC), dimyristoylphosphatidylcholine (DMPC), dioleoylphosphatidylcholine (DOPC), etc.), phosphatidylethanolamines (specifically, distearoylphosphatidylethanolamine (DSPE), dipalmitoylphosphatidylethanolamine (DPPE), dioleoylphosphatidylethanolamine (DOPE), dimyristoylphosphoethanolamine (DMPE), 16:0 monomethyl PE, 16:0 dimethyl PE, 18:1 trans PE, palmitoyloleoyl-phosphatidylethanolamine (POPE), 1-stearoyl-2-oleoyl-phosphatidylethanolamine (SOPE), etc.), glycerophospholipids (specifically, phosphatidylserine, phosphatidic acid, phosphatidylglycerol, phosphatidylinositol, palmitoyloleoylphosphatidylglycerol (POPG), lysophosphatidylcholine, etc.), sphingophospholipids (specifically, sphingomyelin, ceramide phosphoethanolamine, ceramide phosphoglycerol, ceramide phosphoglycerophosphate, etc.), glycerophosphonolipids, sphingophosphonolipids, natural lecithins (specifically, egg yolk lecithin, soybean lecithin, etc.), and hydrogenated phospholipids (specifically, hydrogenated soybean phosphatidylcholine etc.)

Examples of the glyceroglycolipid as the neutral lipid include sulfoxyribosyl glyceride, diglycosyl diglyceride, digalactosyl diglyceride, galactosyl diglyceride, and glycosyl diglyceride.

Examples of the sphingoglycolipid as the neutral lipid include galactosyl cerebroside, lactosyl cerebroside, and ganglioside.

Examples of the sphingoid as the neutral lipid include sphingan, icosasphingan, sphingosine, and derivatives thereof. Examples of the derivatives include those derived from sphingan, icosasphingan, sphingosine, or the like by replacing $-NH_2$ with $-NHCO(CH_2)_xCH_3$ (in the formula, x is an integer of from 0 to 18, and is preferably an integer of 6, 12, or 18).

Examples of the sterol as the neutral lipid include cholesterol, dihydrocholesterol, lanosterol, β-sitosterol, campesterol, stigmasterol, brassicasterol, ergosterol, fucosterol, and 3β-[N-(N',N'-dimethylaminoethyl)carbamoyl]cholesterol (DC-Chol).

The neutral lipid is preferably a phospholipid, a sterol or the like, is more preferably phosphatidylcholine, phosphatidylethanolamine, or cholesterol, and is still more preferably phosphatidylethanolamine, cholesterol, or a combination thereof.

The polymer may be one or more selected from, for example, proteins, albumins, dextran, Polyfect, chitosan, dextran sulfate, and polymers, such as poly-L-lysine, polyethyleneimine, polyaspartic acid, styrene-maleic acid copolymers, isopropylacrylamide-acrylpyrrolidone copolymers, polyethylene glycol-modified dendrimers, polylactic acid, polylactic acid-polyglycolic acid, and PEGylated polylactic acid, and salts thereof.

The salts of the polymer include, for example, a metal salt, an ammonium salt, an acid addition salt, an organic amine addition salt, an amino acid addition salt, and the like. Examples of the metal salt include alkali metal salts such as a lithium salt, a sodium salt and a potassium salt; alkaline earth metal salts such as a magnesium salt and a calcium salt; an aluminum salt; a zinc salt, and the like. Examples of the ammonium salt include salts of ammonium, tetramethylammonium, or the like. Examples of the acid addition salt include inorganic acid salts such as a hydrochloric acid salt, a sulfuric acid salt, a nitric acid salt, and a phosphoric acid salt; and organic acid salts such as an acetic acid salt, a maleic acid salt, a fumaric acid salt, and a citric acid salt. Examples of the organic amine addition salt include addition salts of morpholine, piperidine, or the like. Examples of the amino acid addition salt include addition salts of glycine, phenylalanine, aspartic acid, glutamic acid, lysine, or the like.

The lipid particle of the present invention preferably further contains a water-soluble polymer-conjugated derivative of a lipid or fatty acid. The water-soluble polymer-conjugated derivative of a lipid or fatty acid may be contained in the complex or in the lipid membrane encapsulating the complex. More preferably, the water-soluble polymer-conjugated derivative of a lipid or fatty acid is contained both in the complex and in the lipid membrane.

In cases where the lipid particle of the present invention contains the water-soluble polymer-conjugated derivative of a lipid or fatty acid, the total number of the molecules in the water-soluble polymer-conjugated derivative of a lipid or fatty acid is preferably 0.05 to 0.3 times the total number of the molecules of Compounds (I) and (II), and the cationic lipid other than Compound (I) or (II), and is more preferably 0.07 to 0.25 times, further more preferably 0.1 to 0.2 times the total number of the molecules of Compounds (I) and (II), and the cationic lipid other than Compound (I) or (II).

The water-soluble polymer-conjugated derivative of a lipid or fatty acid is preferably a dual functional substance

of which the molecule contains a moiety having a function to bind to another component of the lipid particle via, for example, hydrophobic affinity, electrostatic interaction, or the like, and also contains another moiety having a function to bind to a solvent used in the production process of the lipid particle via, for example, hydrophilic affinity, electrostatic interaction, or the like.

Examples of the water-soluble polymer-conjugated derivative of a lipid or fatty acid include molecules formed by binding of the neutral lipid listed above as a lipid particle component, Compound (I) or (II), or a fatty acid (e.g., stearic acid, palmitic acid, myristic acid, lauric acid, etc.) to, for example, polyethylene glycol, polyglycerol, polyethyleneimine, polyvinyl alcohol, polyacrylic acid, polyacrylamide, an oligosaccharide, dextrin, a water-soluble cellulose, dextran, chondroitin sulfate, chitosan, polyvinylpyrrolidone, polyaspartic acid amide, poly-L-lysine, mannan, pullulan, an oligoglycerol, or a derivative thereof; and salts of such molecules. More preferred are polyethylene glycol-conjugated derivatives of a lipid or fatty acid, polyglycerol-conjugated derivatives of a lipid or fatty acid, and salts thereof. Still more preferred are polyethylene glycol-conjugated derivatives of a lipid or fatty acid, and salts thereof.

Examples of the polyethylene glycol-conjugated derivatives of a lipid or fatty acid include PEGylated lipids (specifically, polyethylene glycol-phosphatidylethanolamines (more specifically, 1,2-distearoyl-sn-glycero-3-phosphoethanolamine-N-[methoxy( polyethylene glycol)-2000] (PEG-DSPE), 1,2-dimyristoyl-sn-glycero-3-phosphoethanolamine-N-[methoxy (polyethylene glycol)-2000] (PEG-DMPE), etc.), polyoxyethylene hydrogenated castor oil 60, CREMOPHOR EL, etc.), polyethylene glycol sorbitan fatty acid esters (specifically, polyoxyethylene sorbitan monooleate, etc.), and polyethylene glycol fatty acid esters. More preferred are PEGylated lipids, and still more preferred are PEG-DSPE and PEG-DMPE.

Examples of the polyglycerol-conjugated derivatives of a lipid or fatty acid include polyglycerolated lipids (specifically, polyglycerol-phosphatidylethanolamine, etc.) and polyglycerol fatty acid esters. More preferred are polyglycerolated lipids.

Surface modification with, for example, a water-soluble polymer, a polyoxyethylene derivative, etc. may be optionally applied to the lipid particle of the present invention [see ed. D. D. Lasic, F. Martin, Stealth Liposomes, CRC Press Inc., US, 1995, pp. 93-102]. Examples of the polymer that can be used for the surface modification include dextran, pullulan, mannan, amylopectin, and hydroxyethyl starch. Examples of the polyoxyethylene derivative include polysorbate 80, Pluronic F68, polyoxyethylene hydrogenated castor oil 60, polyoxyethylene lauryl alcohol, and PEG-DSPE. The surface modification allows inclusion of the water-soluble polymer-conjugated derivative of a lipid or fatty acid in the complex and in the lipid membrane of the lipid particle.

The average particle diameter of the lipid particle of present invention can be freely selected depending on the needs, but the average particle diameter is preferably adjusted to the diameter described later. The adjustment of the average particle diameter may be achieved by, for example, the extrusion method, a method in which multilamellar liposome vesicles (MLVs) of a large size or other vesicles are mechanically disrupted (in particular, using a Manton-gaulin homogenizer, a microfluidizer, or the like) (see Emulsion and Nanosuspensions for the Formulation of Poorly Soluble Drugs, edited by R. H. Muller, S. Benita and B. Bohm, Scientific Publishers, Stuttgart, Germany, pp. 267-294, 1998), or other methods.

The size, i.e., the average particle diameter of the lipid particle of the present invention is preferably from about 10 nm to 1,000 nm, more preferably from about 30 nm to 300 nm, and still more preferably from about 50 nm to 200 nm.

By administering the composition of the present invention to a mammalian cell, the double-stranded nucleic acid in the composition of the present invention can be introduced into the cell.

The introduction of the composition of the present invention into a mammalian cell in vivo is achieved by a known transfection procedure that can be carried out in vivo. The composition of the present invention is, for example, intravenously administered to a mammal including a human and delivered to, for example, an organ or site affected by a cancer or inflammation, and thereby the double-stranded nucleic acid in the composition of the present invention can be introduced into the cells of the organ or site. The organ or site affected by a cancer or inflammation is not particularly limited. Examples thereof include the stomach, large intestine, pancreas, stomach, small intestine, liver, lung, spleen, kidney, bladder, skin, blood vessels, eye ball, and esophagus. Of these, preferred are the large intestine, pancreas, stomach, small intestine, liver, and esophagus. The composition of the present invention is intravenously administered to a mammal including a human and delivered to, for example, the blood vessels, liver, lung, spleen and/or kidney, and thereby the double-stranded nucleic acid in the composition of the present invention can be introduced into the cells of the organ or the site. The cells of the blood vessels, liver, lung, spleen and/or kidney may be normal cells.

Introduction of the double-stranded nucleic acid of the composition of the present invention into the cells of the organ or the site as the delivery target induces reduction of the expression of the CKAP5 gene in the cells of the organ or the site, thereby treating a CKAP5-associated disease, for example, leukemia, melanoma, a cell tumor, a cancer, a tumor, an adenoma, or the like.

That is, administration of the composition of the present invention to a mammal induces reduction of the expression of the CKAP5 gene, thereby treating a CKAP5-associated disease, for example, leukemia, melanoma, a cell tumor, a cancer, a tumor, an adenoma, or the like. The subject of the administration is preferably a human.

The composition of the present invention can also be used as an analysis tool for the efficacy of suppressing the CKAP5 gene in an in vivo model that is used to evaluate the efficacy of a therapeutic or preventive agent for a cancer.

In cases where the composition of the present invention is used as a therapeutic or preventive agent for a cancer, the administration route is desirably the most effective route for the treatment. Preferred administration routes are an intravenous administration route and an intramuscular administration route, and more preferred is an intravenous administration route.

The dosage varies with the conditions and the age of the subject of the administration, the administration route, and the like. The administration may be performed, for example, at a daily dosage of from about 0.1 μg to 1,000 mg in terms of the double-stranded nucleic acid.

The dosage form suitable for intravenous administration or intramuscular administration may be, for example, an injection. A dispersion of the lipid particle prepared as above can be directly used as a dosage form such an injection. The solvent may be removed from the dispersion before use by means of, for example, filtration, centrifugation, or the like. The dispersion as it is and/or after supplemented with an excipient such as mannitol, lactose, trehalose, maltose, and glycine may be lyophilized before use.

In cases where the dosage form is an injection, the above dispersion of the lipid particle or the composition obtained by solvent removal or lyophilization is preferably mixed with, for example, water, an acid, an alkali, a variety of buffer solutions, physiological saline, an amino acid infusion, or the like to prepare an injection. The injection can also be prepared with the addition of an antioxidant such as citric acid, ascorbic acid, cysteine, and EDTA, an isotonic agent such as glycerol, glucose, and sodium chloride, or the like. The injection can also be lyophilized with the addition of a lyophilization agent such as glycerol.

EXAMPLES

The present invention will be specifically described with reference to the following Examples, Reference Examples and Test Examples, but the present invention is not limited to these Examples and Test Examples.

The proton nuclear magnetic resonance spectra ($^1$H NMR) shown in Reference Examples were measured at 270 MHz, 300 MHz, or 400 MHz. Depending on the compound or the measuring conditions, exchangeable protons may not be clearly observed. The multiplicity of the signal is expressed by usual abbreviations. The symbol "br" indicates an apparently broad signal.

Reference Example 1

Methyl di((9Z,12Z)-octadeca-9,12-dienyl)amine (Compound II-1)

To methylamine (Aldrich, about 2 mol/L solution in tetrahydrofuran, 10.5 mL, 21.0 mmol) was added (9Z,12Z)-octadeca-9,12-dienyl methanesulfonate (Nu-Chek Prep, Inc., 1.03 g, 3.00 mmol). The mixture was heated with stirring at 150°C for 90 minutes using a microwave reaction apparatus. The reaction mixture was diluted with ethyl acetate, and washed with a 2 mol/L sodium hydroxide aqueous solution and then with saturated brine. Drying over anhydrous magnesium sulfate, filtration and concentration under reduced pressure gave a crude product of methyl ((9*Z*,12*Z*)-octadeca-9,12-dienyl)amine.

To the obtained crude product were added (9*Z*,12*Z*)-octadeca-9,12-dienyl methanesulfonate (Nu-Chek Prep, Inc., 0.93 g, 2.70 mmol) and a 50% sodium hydroxide aqueous solution (0.960 g, 12.0 mmol). The mixture was heated with stirring at 135°C for 60 minutes on an oil bath. After allowed to cool back to room temperature, the reaction mixture was diluted with ethyl acetate, and washed with water and then with saturated brine. Drying over anhydrous magnesium sulfate, filtration and concentration under reduced pressure were performed. The residue was purified by silica gel column chromatography (chloroform/methanol = 100/0 to 97/3) to give Compound II-1 (1.07 g, 67.2%).
ESI-MS m/z: 529 $(M+H)^+$; $^1$H-NMR ($CDCl_3$) δ: 0.89 (t, *J* = 6.7 Hz, 6H), 1.29 (br s, 32H), 1.40-1.51 (m, 4H), 1.97-2.06 (m, 8H), 2.20 (s, 3H), 2.30 (t, *J* = 7.6 Hz, 4H), 2.77 (t, *J* = 5.8 Hz, 4H), 5.28-5.43 (m, 8H).

Reference Example 2

Methyl di((*Z*)-hexadec-9-enyl)amine (Compound II-2)

Compound II-2 (0.491 g, 51.6%) was produced in the same manner as in Reference Example 1, using methylamine (Aldrich, about 2 mol/L solution in tetrahydrofuran, 10.0 mL, 20.0 mmol) and (Z)-hexadec-9-enyl methanesulfonate (Nu-Chek Prep, Inc., 1.21 g, 3.80 mmol).
ESI-MS m/z: 477 $(M+H)^+$; $^1$H-NMR ($CDCl_3$) δ: 0.88 (t, *J* = 6.7 Hz, 6H), 1.29 (br s, 36H), 1.46-1.57 (m, 4H), 1.97-2.05 (m, 8H), 2.33 (s, 3H), 2.45 (t, *J* = 7.9 Hz, 4H), 5.29-5.41 (m, 4H).

Reference Example 3

Methyl di (11*Z*, 14*Z*)-icosa-11,14-dienyl)amine (Compound II-3)

Compound II-3 (1.27 g, 54.4%) was produced in the same manner as in Reference Example 1, using methylamine (Aldrich, about 2 mol/L solution in tetrahydrofuran, 16.0 mL, 32.0 mmol) and (11*Z*,14*Z*)-icosa-11,14-dienyl methanesulfonate (Nu-Chek Prep, Inc., 2.98 g, 8.00 mmol).
ESI-MS m/z: 585 $(M+H)^+$; $^1H$-NMR ($CDCl_3$) $\delta$: 0.89 (t, *J* = 6.7 Hz, 6H), 1.27 (br s, 40H), 1.39-1.51 (m, 4H), 2.01-2.09 (m, 8H), 2.20 (s, 3H), 2.30 (t, *J* = 7.6 Hz, 4H), 2.79 (d, *J* = 6.3 Hz, 4H), 5.28-5.43 (m, 8H).

Reference Example 4

Di((9Z,12Z)-octadeca-9,12-dienyl)amine (Compound II-4)

Compound II-4 (0.838 g, 36.2%) was produced in the same manner as in Reference Example 1, using ammonia (Tokyo Chemical Industry Co., Ltd., about 2 mol/L solution in methanol, 18.0 mL, 36.0 mmol) and (9Z,12Z)-octadeca-9,12-dienyl methanesulfonate (Nu-Chek Prep, Inc., 2.79 g, 8.10 mmol). ESI-MS m/z: 515 $(M+H)^+$; $^1H$-NMR ($CDCl_3$) $\delta$: 0.89 (t, *J* = 6.9 Hz, 6H), 1.30 (br s, 33H), 1.41-1.54 (m, 4H), 2.01-2.09 (m, 8H), 2.59 (t, *J* = 7.2 Hz, 4H), 2.77 (d, *J* = 5.6 Hz, 4H), 5.28-5.43 (m, 8H).

Reference Example 5

Di((Z)-octadec-9-enyl)amine (Compound II-5)

Compound II-5 (0.562 g, 36.2%) was produced in the same manner as in Reference Example 1, using ammonia (Tokyo Chemical Industry Co., Ltd., about 2 mol/L solution in methanol, 12.0 mL, 24.0 mmol) and (Z)-octadec-9-enyl methanesulfonate (Nu-Chek Prep, Inc., 1.87 g, 5.40 mmol).
ESI-MS m/z: 519 $(M+H)^+$; $^1H$-NMR ($CDCl_3$) $\delta$: 0.88 (t, *J* = 6.7 Hz, 6H), 1.29 (br s, 45H), 1.41-1.52 (m, 4H), 1.97-2.05 (m, 8H), 2.58 (t, *J* = 7.2 Hz, 4H), 5.28-5.40 (m, 4H).

Reference Example 6

Methyl di((Z)-octadec-9-enyl)amine (Compound II-6)

Compound II-6 (1.20 g, 70. 2%) was produced in the same manner as in Reference Example 1, using methylamine (Aldrich, about 2 mol/L solution in tetrahydrofuran, 11.2 mL, 22.4 mmol) and (Z)-octadec-9-enyl methanesulfonate (Nu-Chek Prep, Inc., 2.11 g, 6.09 mmol).
ESI-MS m/z: 533 $(M+H)^+$; $^1H$-NMR ($CDCl_3$) $\delta$: 0.88 (t, *J* = 6.6 Hz, 6H), 1.27 (br s, 44H), 1.39-1.50 (m, 4H), 1.97-2.06 (m, 8H), 2.20 (s, 3H), 2.30 (t, *J* = 7.6 Hz, 4H), 5.28-5.40 (m, 4H).

Reference Example 7

Trans-1-(tert-butoxycarbonyl)-3,4-bis(((*Z*)-octadec-9-enoylo xy)methyl)pyrrolidine (Compound VII-1)

In dichloromethane (6 mL) was dissolved tert-butyl trans-3,4-bis(hydroxymethyl)pyrrolidine-1-carboxylate (156 mg, 0.674 mmol) synthesized with reference to WO 2006/100036. To the solution were added oleic acid (Tokyo Chemical Industry Co., Ltd., 419 mg, 1.48 mmol), water-soluble carbodiimide (WSCD) (Kokusan Chemical Co., Ltd., 297 mg, 1.55 mmol) and 4-dimethylaminopyridine (DMAP) (Tokyo Chemical Industry Co., Ltd., 20.6 mg, 0.169 mmol). The mixture was stirred at room temperature overnight. To the reaction mixture, saturated sodium hydrogen carbonate aqueous solution was added, followed by extraction with ethyl acetate. The organic layer was washed with water and saturated brine, dried over anhydrous magnesium sulfate, and concentrated under reduced pressure. The residue was purified by silica gel column chromatography (hexane/chloroform= 50/50 to 0/100) to give Compound VII-1 (280 mg, 54.6%).
ESI-MS m/z: 761 $(M+H)^+$; $^1H$-NMR ($CDCl_3$) $\delta$: 0.88 (t, *J* = 6.6 Hz, 6H), 1.25-1.46 (m, 36H), 1.46 (s, 9H), 1.46-1.66 (m, 8H), 1.97-2.04 (m, 8H), 2.27-2.38 (m, 6H), 3.10-3.23 (m, 2H), 3.53-3.66 (m, 2H), 4.03 (dd, *J* = 10.8, 6.0 Hz, 2H), 4.14 (dd, *J* = 10.8, 6.0 Hz, 2H), 5.28-5.40 (m, 4H).

Reference Example 8

Trans-1-(tert-butoxycarbonyl)-3,4-bis(((9*Z*,12*Z*)-octadeca-9, 12-dienoyloxy)methyl)pyrrolidine (Compound VII-2)

Compound VII-2 (351 mg, 71.7%) was produced in the same manner as in Reference Example 7, using tert-butyl trans-3,4-bis(hydroxymethyl)pyrrolidine-1-carboxylate (150 mg, 0.674 mmol) synthesized with reference to WO 2006/100036 and linoleic acid (Aldrich, 400 mg, 1.48 mmol).
ESI-MS m/z: 757 $(M+H)^+$; $^1$H-NMR ($CDCl_3$) $\delta$: 0.89 (t, *J* = 6.8 Hz, 6H), 1.21-1.45 (m, 26H), 1.46 (s, 9H), 1.47-1.68 (m, 6H), 2.05 (q, *J* = 6.7 Hz, 8H), 2.26-2.38 (m, 6H), 2.77 (t, *J* = 5.9 Hz, 4H), 3.10-3.23 (m, 2H), 3.53-3.66 (m, 2H), 4.03 (dd, *J* = 11.0, 6.0 Hz, 2H), 4.14 (dd, *J* =11.0, 6.0 Hz, 2H), 5.28-5.43 (m, 8H).

Reference Example 9

Trans-3,4-bis(((Z)-octadec-9-enoyloxy)methyl)pyrrolidine (Compound I-1)

In dichloromethane (6 mL) was dissolved Compound VII-1 (278 mg, 0.366 mmol) produced in Reference Example 7. To the solution was added trifluoroacetic acid (0.563 mL, 7.31 mmol). The mixture was stirred at room temperature for 3 hours. To the reaction mixture, a saturated sodium hydrogen carbonate aqueous solution was added, and the aqueous layer was extracted with chloroform. The organic layer was washed with saturated brine, dried over anhydrous magnesium sulfate, filtered and concentrated under reduced pressure. The residue was dissolved in a small amount of methanol, and the solution was adsorbed onto BONDESIL-SCX (Varian Medical Systems Inc., 6 g) packed in a plastic column. The column was washed with methanol, and the substance of interest was eluted with an ammonia/methanol solution (Tokyo Chemical Industry Co., Ltd., 2 mol/L). The fraction containing the substance of interest was concentrated under reduced pressure to give Compound I-1 (162 mg, 67.2%.
ESI-MS m/z: 661 $(M+H)^+$; $^1$H-NMR ($CDCl_3$) $\delta$: 0.88 (t, *J* = 6.6 Hz, 6H), 1.27-1.35 (m, 40H), 1.56-1.64 (m, 4H), 2.01 (q, *J* = 5.9 Hz, 8H), 2.09-2.16 (m, 2H), 2.30 (t, *J* = 7.5 Hz, 4H), 2.72 (dd, *J* = 11.3, 5.5 Hz, 2H), 3. 11 (dd, *J* = 11.3, 7. 1 Hz, 2H), 3.99-4.12 (m, 4H), 5.29-5.40 (m, 4H).

Reference Example 10

Trans-3,4-bis(((9*Z*,12*Z*)-octadeca-9,12-dienoyloxy)methyl)pyr rolidine (Compound I-2)

Compound I-2 (224 mg, 73.6%) was produced in the same manner as in Reference Example 9, using Compound VII-2 (350 mg, 0.463 mmol) produced in Reference Example 8.
ESI-MS m/z: 657 $(M+H)^+$; $^1$H-NMR ($CDCl_3$) $\delta$: 0.89 (t, *J* = 6.8 Hz, 6H), 1.26-1.40 (m, 28H), 1.57-1.66 (m, 4H), 2.05 (q, *J* = 6.6 Hz, 8H), 2.09-2.17 (m, 2H), 2.31 (t, *J* = 7.5 Hz, 4H), 2.72 (dd, *J* = 11.3, 6.0 Hz, 2H), 2.77 (t, *J* = 6.2 Hz, 4H), 3.11 (dd, *J* = 11.3, 7.3 Hz, 2H), 3.99-4.13 (m, 4H), 5.28-5.43 (m, 8H).

Reference Example 11

Trans-1-methyl-3,4-bis(((9*Z*,12*Z*)-octadeca-9,12-dienoyloxy)m ethyl)pyrrolidine (Compound I-3)

In 1,2-dichloroethane (1.5 mL) and methanol (1.5 mL) was dissolved Compound I-2 (80 mg, 0.12 mmol) produced in Reference Example 10. To the solution were added formaldehyde (0.091 mL, 1.22 mmol) and sodium triacetoxyborohydride (Acros Organics, 129 mg, 0.610 mmol) in several portions. The mixture was stirred at room temperature for 1.5 hours. To the reaction mixture, a saturated sodium hydrogen carbonate aqueous solution was added, and the aqueous layer was extracted with ethyl acetate. The organic layer was washed with saturated brine, dried over anhydrous magnesium sulfate, filtered and concentrated under reduced pressure. The residue was purified by silica gel column chromatography (chloroform/methanol = 100/0 to 93/7) to give Compound I-3 (66 mg, 81%).
ESI-MS m/z: 671 $(M + H)^+$; $^1$H-NMR ($CDCl_3$) $\delta$: 0.89 (t, *J*= 6.8 Hz, 6H), 1.25-1.40 (m, 28H), 1.57-1.66 (m, 4H), 2.05 (q, *J* = 6.7 Hz, 8H), 2.13-2.24 (m, 2H), 2.27-2.37 (m, 9H), 2.66 (dd, *J* = 9.2, 7.3 Hz, 2H), 2.77 (t, *J* = 5.7 Hz, 4H), 3.99-4.12 (m, 4H), 5.28-5.43 (m, 8H).

Reference Example 12

Trans-1-methyl-3,4-bis(((Z)-octadec-9-enoyloxy)methyl)pyrro lidine (Compound I-4)

Compound I-4 (47 mg, 92%) was produced in the same manner as in Reference Example 11, using Compound

I-1 (50 mg, 0.076 mmol) produced in Reference Example 9.
ESI-MS m/z: 675 $(M+H)^+$; $^1$H-NMR ($CDCl_3$) δ: 0.88 (t, *J* = 6.6 Hz, 6H), 1.26-1.35 (m, 40H), 1.56-1.65 (m, 4H), 2.01 (q, *J* = 5.5 Hz, 8H), 2.15-2.24 (m, 2H), 2.27-2.37 (m, 9H), 2.67 (dd, *J* = 9.3, 7.1 Hz, 2H), 3.99-4.12 (m, 4H), 5.29-5.40 (m, 4H).

Example 1

Formulations were produced in the following manner using Compound II-1 obtained in Reference Example 1, Compound I-3 obtained in Reference Example 11 and each of siRNAs A and B in Table 3.

Each of the double-stranded nucleic acids was dissolved in distilled water to a concentration of 24 mg/mL before use (hereinafter referred to as an siRNA solution).

Compound II-1 and PEG-DSPE Na were suspended at a ratio of 57.3:5.52 in terms of mmol/L in an aqueous solution containing hydrochloric acid and ethanol. The suspension was subjected to the repetition of vortex mixing and heating to prepare a homogenous suspension. The suspension was passed through a 0.2-μm polycarbonate membrane filter and then through a 0.05-μm polycarbonate membrane filter at room temperature to prepare a dispersion of lead particles. The average particle diameter of the lead particles was determined with a particle size analyzer (Zetasizer Nano ZS, Malvern Instruments) to be within the range of from 30 nm to 100 nm. The dispersion of lead particles was mixed with the siRNA solution at a ratio of 3:1 (= the dispersion of lead particles:the siRNA solution). The mixture was further mixed with a three-fold volume of distilled water to prepare a dispersion of particles of a complex of the cationic lipids and the double-stranded nucleic acid.

Separately, Compound II-1, Compound I-3, PEG-DSPE Na, DSPC and cholesterol were weighed and then dissolved in 90 vol% ethanol at a ratio of 2.98:5.97:2.94:5.71:11.8 in terms of mmol/L to prepare a solution of lipid membrane components.

The solution of lipid membrane components was heated and then mixed with the dispersion of particles of a complex of the cationic lipids and the double-stranded nucleic acid at a ratio of 1:1. The mixture was further mixed with a several-fold volume of distilled water to give a crude formulation.

The crude formulation was concentrated using Amicon Ultra (Millipore Corporation). The solvent was replaced by physiological saline, and then filtered through a 0.2-μm filter (Toyo Roshi Kaisha, Ltd.) in a clean bench. The siRNA concentration of the formulation was measured, and the formulation was diluted with physiological saline to an siRNA concentration of 1.0 mg/mL to give Formulation 1-A or 1-B (a composition containing Compound II-1 and Compound I-3 as the lipids and containing siRNA A or B as the double-stranded nucleic acid). The average particle diameter of the lipid particles in each of the formulations was measured with the particle size analyzer. The results are shown in Table 4.

Table 4

| Formulation | 1-A | 1-B |
|---|---|---|
| siRNA | A | B |
| Diameter of particles in formulation (nm) | 86 | 86 |

Example 2

Formulations 1-C to 1-F (compositions containing Compound II-1 and Compound I-3 as the lipids and containing any one of siRNAs C to F as the double-stranded nucleic acid) were produced in the same manner as in Example 1, using Compound II-1 obtained in Reference Example 1, Compound I-3 obtained in Reference Example 11 and each of siRNAs C to F in Table 3. The average particle diameter was measured with the particle size analyzer. The results are shown in Table.5.

Table 5

| Formulation | 1-C | 1-D | 1-E | 1-F |
|---|---|---|---|---|
| siRNA | C | D | E | F |
| Diameter of particles in formulation (nm) | 81 | 80 | 83 | 83 |

Example 3

Formulations 1-B', 1-D', 1-E' and 1-G (compositions containing Compound II-1 and Compound I-3 as the lipids and containing siRNA B, D, E or G as the double-stranded nucleic acid) were produced in the same manner as in

Example 1, using Compound II-1 obtained in Reference Example 1, Compound I-3 obtained in Reference Example 11 and each of siRNAs B, D, E and G in Table 3. The average particle diameter was measured with the particle size analyzer. The results are shown in Table 6.

Table 6

| Formulation | 1-B' | 1-D' | 1-E' | 1-G |
|---|---|---|---|---|
| siRNA | B | D | E | G |
| Diameter of particles in formulation (nm) | 84 | 84 | 86 | 83 |

Example 4

Formulations 1-A' and 1-B" (compositions containing Compound II-1 and Compound I-3 as the lipids and containing siRNA A or B as the double-stranded nucleic acid) were produced in the same manner as in Example 1, using Compound II-1 obtained in Reference Example 1, Compound I-3 obtained in Reference Example 11 and each of siRNA A and B in Table 3. The average particle diameter was measured with the particle size analyzer. The results are shown in Table 7.

Table 7

| Formulation | 1-A' | 1-B" |
|---|---|---|
| siRNA | A | B |
| Diameter of particles in formulation (nm) | 85 | 85 |

Example 5

Formulations 1-B''', 1-C'', 1-D'', 1-E'' and 1-F'' (compositions containing Compound II-1 and Compound I-3 as the lipids and containing siRNA B, C, D, E or F as the double-stranded nucleic acid) were produced in the same manner as in Example 1, using Compound II-1 obtained in Reference Example 1, Compound I-3 obtained in Reference Example 11 and each of siRNAs B, C, D, E and F in Table 3. The average particle diameter was measured with the particle size analyzer. The results are shown in Table 8.

Table 8

| Formulation | 1-B''' | 1-C" | 1-D" | 1-E" | 1-F" |
|---|---|---|---|---|---|
| siRNA | B | C | D | E | F |
| Diameter of particles in formulation (nm) | 78 | 79 | 79 | 80 | 81 |

Test Example 1

In vivo mRNA knockdown evaluation test was performed using each of Formulations 1-A and 1-B of Example 1 as follows.

Human pancreatic cancer cell line MIA PaCa-2 was obtained from JCRB Cell Bank. MIA PaCa-2 cells were cultured in high glucose DMEM (GIBCO, 11995-065) supplemented with 10% inactivated fetal calf serum (GIBCO) and 1 vol% penicillin-streptomycin (GIBCO, 15140-122) in 5% $CO_2$ at 37°C. The cells were suspended in phosphate buffered saline (PBS) at $1.6 \times 10^8$ cells/mL. A 50 μL aliquot of the cell suspension was transplanted into the dorsal skin of each of SCID mice (purchased from CLEA Japan, Inc.) ($8 \times 10^6$ cells in 0.05 mL phosphate buffered saline (PBS) per animal). The mice were divided into groups of five depending on the tumor volume. Each of the formulations obtained in Example 1 was intravenously administered to the mice in an amount equivalent to 10 mg/kg of the siRNA. For a saline administration group, physiological saline was administered at a dose of 10 mL/kg. The body weights of the mice were measured before and 48 hours after the administration. After the measurement of the weights, the mice were euthanized and the subcutaneous tumors were harvested. The tumors were immediately frozen in liquid nitrogen and stored at -80°C until use.

Each of the tumor samples was placed in a 2-mL round bottom tube, and 0.5 mL of Trizol reagent (Invitrogen, 10296-028) and zirconia balls (AS ONE Corporation, 5-4060-13) of 5 mm in diameter were added to the tube. The sample was disrupted with TissueLyser II (QIAGEN) under the conditions of 1/25 freq and 1.5 minutes × twice. The

disrupted sample was centrifuged (at 10,000 rpm for 10 minutes), and the supernatant was recovered. To the supernatant, 200 μL of chloroform was added, and the mixture was vigorously shaken and centrifuged again (at 15,000 rpm for 15 minutes). From a 200 μL of the supernatant, RNA was extracted using the high-throughput system for extraction of nucleic acids, MagNA Pure 96 (Roche, 5195322), and Cellular RNA Large Volume Kit (Roche, 5467535) in accordance with the manufacturer's instructions. The concentration of the extracted RNA was measured with a DropSense96 absorption photometer (Trinean). About 500 to 1,000 ng of RNA was reverse transcribed with Transcriptor (Roche, 4897030). The reaction reagents and the reaction conditions were as described in the manufacturer's instructions attached to Transcriptor. The obtained cDNA sample was diluted with $dH_2O$ to ten-fold and used as a template for quantitative PCR (qPCR). The qPCR was performed using TaqMan Gene Expression Master Mix (Applied Biosystems, 4369542) and TaqMan Gene Expression Assays (Applied Biosystems, 4331182). The PCR conditions were as described in the manufacturer's instructions attached to TaqMan Gene Expression. The CKAP5 mRNA level of the sample was calculated as a relative value, with the CKAP5 mRNA level in the saline administration group taken as 1.

Fig. 1 shows the CKAP5 mRNA level in the tumors.

As is clear from Fig. 1, comparison with the saline administration group revealed that the formulations obtained in Example 1 suppressed the expression of the CKAP5 gene.

Test example 2

In vivo mRNA knockdown evaluation test was performed in the same manner as in Test Example 1, using each of Formulations 1-C to 1-F obtained in Example 2.

Fig. 2 shows the CKAP5 mRNA level in the tumors.

As is clear from Fig. 2, comparison with the saline administration group revealed that the formulations obtained in Example 2 suppressed the expression of the CKAP5 gene.

Test Example 3

In vivo mRNA knockdown evaluation test was performed using each of Formulations 1-D and 1-E of Example 2 as follows.

Human pancreatic cancer cell line MIA PaCa-2 was obtained from JCRB Cell Bank. MIA PaCa-2 cells were cultured in high glucose DMEM (GIBCO, 11995-065) supplemented with 10% inactivated fetal calf serum (GIBCO) and 1 vol% penicillin-streptomycin (GIBCO, 15140-122) in 5% $CO_2$ at 37°C. The cells were suspended in phosphate buffered saline (PBS) at $1.6 \times 10^8$ cells/mL. A 50 μL aliquot of the cell suspension was transplanted into the dorsal skin of each of SCID mice (purchased from CLEA Japan, Inc.) ($8 \times 10^6$ cells in 0.05 mL PBS per animal). The mice were divided into groups of five depending on the tumor volume. Each of the formulations obtained in Example 2 was intravenously administered to the mice in an amount equivalent to 10 mg/kg of the siRNA. For a saline administration group, physiological saline was administered at a dose of 10 mL/kg. The body weights of the mice were measured before and 48 hours after the administration. After the measurement of the weights, the mice were euthanized and the subcutaneous tumors and the bone marrow cells in the cavity of the femur were harvested. The tumors and the bone marrow cells were immediately frozen in liquid nitrogen and stored at -80°C until use.

RNA was extracted from each of the tumor samples, and cDNA was obtained from the extracted RNA in the same manner as in Test Example 1 and then used as a template for qPCR as in Test Example 1. The qPCR was performed using TaqMan Gene Expression Master Mix (Applied Biosystems, 4369542). The primers and probes for the human or mouse genes were obtained from TaqMan Gene Expression Assays (Applied Biosystems, 4331182). The PCR conditions were as described in the manufacturer' s instructions attached to TaqMan Gene Expression. The human or mouse CKAP5 mRNA level of the sample was calculated as a relative value, with the human or mouse CKAP5 mRNA level in the saline administration group taken as 1.

Fig. 3 shows the human CKAP5 mRNA level in the tumors.

Fig. 4 shows the mouse CKAP5 mRNA level in the bone marrow cells.

As is clear from Figs. 3 and 4, comparison with the saline administration groups revealed that the formulations obtained in Example 2 suppressed the expression of the CKAP5 gene in the tumors, but hardly suppressed the expression of the CKAP5 gene in the normal bone marrow.

Test Example 4

Tumor growth evaluation test was performed using each of Formulations 1-B', 1-D', 1-E'and 1-G of Example 3 as follows.

The test was performed using a xenograft model generated by transplanting the human pancreatic cancer cell line MIA PaCa-2 into SCID mice in the same manner as in Test Example 1. The mice were divided into groups of six

depending on the tumor volume (Day 0). Each of the formulations obtained in Example 3 was intravenously administered in an amount equivalent to 10 mg/kg of the siRNA to the mice on Day 0 and Day 7. For a control group, physiological saline was administered at a dose of 10 mL/kg. The tumor diameter of each animal was measured on Day 0 to Day 21, and the tumor volume and the volume ratio were calculated according to the following equations.

$$\text{Tumor volume (mm}^3\text{)} = \text{length (mm)} \times \text{width (mm)} \times \text{width (mm)} \times 0.5$$

$$\text{Volume ratio (V/V0)} = \text{tumor volume at each point of time (mm}^3\text{)/tumor volume on day 0 (mm}^3\text{)}$$

Figs. 5 to 8 show the transition of the relative tumor volume.

As is clear from Figs. 5 to 8, comparison with the saline administration group in the in vivo efficacy evaluation test revealed that the formulations obtained in Example 3 had strong antitumor action.

The results showed that administration of the composition of the present invention to a mammal reduces the expression of the CKAP5 gene in the living body, thereby treating a CKAP5-associated disease.

Test Example 5

Tumor growth evaluation test was performed using each of Formulations 1-A' and 1-B" of Example 4 as follows.

The test was performed using a xenograft model generated by transplanting the human pancreatic cancer cell line MIA PaCa-2 into SCID mice in the same manner as in Test Example 1. The mice were divided into groups of six depending on the tumor volume (Day 0). Each of the formulations obtained in Example 4 was intravenously administered in an amount equivalent to 10 mg/kg of the siRNA to the mice on Day 0 and Day 7. For a control group, physiological saline was administered at a dose of 10 mL/kg. The tumor diameter of each animal was measured on Day 0 to Day 21, and the tumor volume and the volume ratio were calculated in the same manner as in Test Example 4. Fig. 9 shows the transition of the relative tumor volume.

As is clear from Fig. 9, comparison with the saline administration group in the in vivo efficacy evaluation test revealed that the formulations obtained in Example 4 had strong antitumor action.

The results showed that administration of the composition of the present invention to a mammal reduces the expression of the CKAP5 gene in the living body, thereby treating a CKAP5-associated disease.

Test Example 6

Tumor growth evaluation test was performed using each of Formulations 1-B'", 1-C", 1-D", 1-E" and 1-F'' of Example 5 as follows.

The test was performed using a xenograft model generated by transplanting the human pancreatic cancer cell line MIA PaCa-2 into SCID mice in the same manner as in Test Example 1. The mice were divided into groups of six depending on the tumor volume (Day 0). Each of the formulations obtained in Example 5 was intravenously administered in an amount equivalent to 10 mg/kg of the siRNA to the mice on Day 0 and Day 7. For a control group, physiological saline was administered at a dose of 10 mL/kg. The tumor diameter of each animal was measured on Day 0 to Day 21, and the tumor volume and the volume ratio were calculated in the same manner as in Test Example 4. Figs. 10 and 11 show the transition of the relative tumor volume.

As is clear from Figs. 10 and 11, comparison with the saline administration group in the in vivo efficacy evaluation test revealed that the formulations obtained in Example 5 had strong antitumor action.

The results showed that administration of the composition of the present invention to a mammal reduces the expression of the CKAP5 gene in the living body, thereby treating a CKAP5-associated disease.

INDUSTRIAL APPLICABILITY

Administration of the composition of the present invention to a mammal suppresses the expression of the CKAP5 gene in the living body, thereby treating a CKAP5-associated disease.

SEQUENCE LISTING FREE TEXT

SEQ ID NO: 1 target in CKAP5 mRNA
SEQ ID NO: 2 target in CKAP5 mRNA
SEQ ID NO: 3 target in CKAP5 mRNA
SEQ ID NO: 4 target in CKAP5 mRNA
SEQ ID NO: 5 target in CKAP5 mRNA
SEQ ID NO: 6 target in CKAP5 mRNA
SEQ ID NO: 7 siRNA sense
SEQ ID NO: 8 siRNA antisense
SEQ ID NO: 9 siRNA sense
SEQ ID NO: 10 siRNA antisense
SEQ ID NO: 11 siRNA sense
SEQ ID NO: 12 siRNA antisense
SEQ ID NO: 13 siRNA sense
SEQ ID NO: 14 siRNA antisense
SEQ ID NO: 15 siRNA sense
SEQ ID NO: 16 siRNA antisense
SEQ ID NO: 17 siRNA sense
SEQ ID NO: 18 siRNA antisense
SEQ ID NO: 19 siRNA sense
SEQ ID NO: 20 siRNA antisense

## Claims

**1.** A composition comprising a lipid particle containing a double-stranded nucleic acid as a drug and a cationic lipid, the double-stranded nucleic acid having a sense strand and an antisense strand, the sense and antisense strands having at least 25 base pairs, the antisense strand having a base sequence complementary to the sequence of at least 19 contiguous bases in CKAP5 gene mRNA of any one of SEQ ID NOs: 1 to 6 and having a maximum length of 35 nucleotides,
the cationic lipid being represented by formula (I):

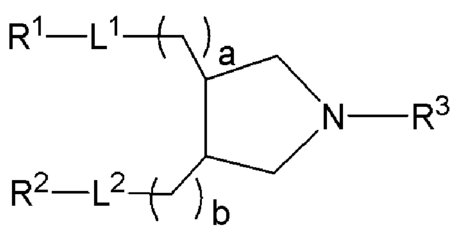

(I)

,

wherein

$R^1$ and $R^2$ are the same or different and are each linear or branched alkyl, alkenyl or alkynyl of 12 to 24 carbon atoms;
$L^1$ and $L^2$ are the same or different and are each -CO-O- or -O-CO-;
a and b are the same or different and are each 1 to 3; and

$R^3$ is a hydrogen atom, alkyl of 1 to 6 carbon atoms, or alkenyl of 3 to 6 carbon atoms.

**2.** The composition according to claim 1, wherein the lipid particle further contains a cationic lipid represented by formula (II):

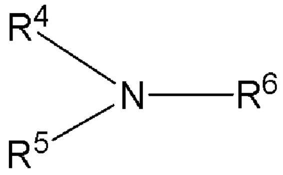

(II)

,

wherein

$R^4$ and $R^5$ are the same or different and are each linear or branched alkyl, alkenyl or alkynyl of 12 to 24 carbon atoms; and

$R^6$ is a hydrogen atom, alkyl of 1 to 6 carbon atoms, alkenyl of 3 to 6 carbon atoms, pyrrolidin-3-yl, piperidin-3-yl, piperidin-4-yl, alkyl of 1 to 6 carbon atoms substituted with a substituent, or alkenyl of 3 to 6 carbon atoms substituted with a substituent, and each substituent is one to three of the same or different groups selected from amino, monoalkylamino, dialkylamino, hydroxy, alkoxy, carbamoyl, monoalkylcarbamoyl, dialkylcarbamoyl, pyrrolidinyl, piperidyl, and morpholinyl.

**3.** The composition according to claim 2, wherein $R^4$ and $R^5$ are the same and are dodecyl, tridecyl, tetradecyl, 2,6,10-trimethylundecyl, pentadecyl, 3,7,11-trimethyldodecyl, hexadecyl, heptadecyl, octadecyl, 6,10,14-trimethylpentadecan-2-yl, nonadecyl, 2,6,10,14-tetramethylpentadecyl, icosyl, 3,7,11,15-tetramethylhexadecyl, henicosyl, docosyl, tricosyl, tetracosyl, (*Z*)-tetradec-9-enyl, (*Z*)-hexadec-9-enyl, (*Z*)-octadec-6-enyl, (*Z*)-octadec-9-enyl, (*E*)-octadec-9-enyl, (*Z*)-octadec-11-enyl, (9*Z*,12*Z*)-octadeca-9,12-dienyl, (9*Z*,12*Z*,15*Z*)-octadeca-9,12,15-trienyl, (*Z*)-icos-11-enyl, (11*Z*,14*Z*)-icosa-11,14-dienyl, 3,7,11-trimethyldodeca-2,6,10-trienyl, or 3,7,11,15-tetramethylhexadec-2-enyl.

**4.** The composition according to claim 2, wherein $R^4$ and $R^5$ are the same and are tetradecyl, hexadecyl, (*Z*)-hexadec-9-enyl, (*Z*)-octadec-6-enyl, (*Z*)-octadec-9-enyl, (9*Z*,12*Z*)-octadeca-9,12-dienyl, (*Z*)-icos-11-enyl, or (11*Z*,14*Z*)-icosa-11,14-dienyl.

**5.** The composition according to claim 3 or 4, wherein $R^6$ is a hydrogen atom, methyl, pyrrolidin-3-yl, piperidin-3-yl, piperidin-4-yl, alkyl of 1 to 6 carbon atoms substituted with a substituent, or alkenyl of 3 to 6 carbon atoms substituted with a substituent, and each substituent is one to three of the same or different groups selected from amino, monoalkylamino, dialkylamino, hydroxy, alkoxy, carbamoyl, monoalkylcarbamoyl, dialkylcarbamoyl, pyrrolidinyl, piperidyl, and morpholinyl.

**6.** The composition according to any one of claims 1 to 5, wherein $R^3$ is a hydrogen atom or methyl.

**7.** The composition according to any one of claims 1 to 6, wherein $L^1$ and $L^2$ are each -O-CO-; and $R^1$ and $R^2$ are the same and are dodecyl, tetradecyl, hexadecyl, octadecyl, icosyl, docosyl, tetracosyl, (*Z*)-tetradec-9-enyl, (*Z*)-hexadec-9-enyl, (*Z*)-octadec-6-enyl, (*Z*)-octadec-9-enyl, (*E*)-octadec-9-enyl, (*Z*)-octadec-11-enyl, (9*Z*,12*Z*)-octadeca-9,12-dienyl, (9*Z*,12*Z*,15*Z*)-octadeca-9,12,15-trienyl, (*Z*)-icos-11-enyl, (11*Z*,14*Z*)-icosa-11,14-dienyl, 3,7,11-trimethyldodeca-2,6,10-trienyl, or 3,7,11,15-tetramethylhexadec-2-enyl.

**8.** The composition according to any one of claims 1 to 6, wherein $L^1$ and $L^2$ are each -CO-O-; and $R^1$ and $R^2$ are the same and are tridecyl, pentadecyl, heptadecyl, nonadecyl, henicosyl, tricosyl, (*Z*)-tridec-8-enyl, (*Z*)-pentadec-8-enyl, (*Z*)-heptadec-5-enyl, (*Z*)-heptadec-8-enyl, (*E*)-heptadec-8-enyl, (*Z*)-heptadec-10-enyl, (8*Z*,11*Z*)-heptadeca-8,11-dienyl, (8*Z*,11*Z*,14*Z*)-heptadeca-8,11,14-trienyl, (*Z*)-nonadec-10-enyl, (10*Z*,13*Z*)-nonadeca-10,13-dienyl, (11*Z*,14*Z*)-icosa-11,14-dienyl, 2,6,10-trimethylundeca-1,5,9-trienyl, or 2,6,10,14-tetramethylpentadec-1-enyl.

**9.** The composition according to any one of claims 1 to 8, wherein the sense and antisense strands of the double-stranded nucleic acid contained as a drug are of SEQ ID NOs: 7 and 8, SEQ ID NOs: 9 and 10, SEQ ID NOs: 11 and 12, SEQ ID NOs: 13 and 14, SEQ ID NOs: 15 and 16, SEQ ID NOs: 17 and 18, or SEQ ID NOs: 19 and 20, respectively.

10. The composition according to any one of claims 1 to 9, wherein the cationic lipid forms a complex with the double-stranded nucleic acid, or the cationic lipid combined with a neutral lipid and/or a polymer forms a complex with the double-stranded nucleic acid.

11. The composition according to any one of claims 1 to 9, wherein the cationic lipid forms a complex with the double-stranded nucleic acid, or the cationic lipid combined with a neutral lipid and/or a polymer forms a complex with the double-stranded nucleic acid, and wherein the lipid particle is formed of the complex and a lipid membrane encapsulating the complex.

12. A method for suppressing the expression of a CKAP5 gene, the method comprising introducing the double-stranded nucleic acid into a cell by using the composition according to any one of claims 1 to 11.

13. The method according to claim 12, wherein the cell is a cell present in a tumor of a mammal.

14. The method according to claim 12, wherein the cell is a cell present in the large intestine, pancreas, stomach, small intestine, liver, or esophagus of a mammal.

15. The method according to any one of claims 12 to 14, wherein the introduction into the cell is achieved by intravenous administration.

16. A method for treating a CKAP5-associated disease, the method comprising administering the composition according to any one of claims 1 to 11 to a mammal.

17. The method according to claim 16, wherein the administration is intravenous administration.

18. A method for treating a cancer, the method comprising administering the composition according to any one of claims 1 to 11 to a mammal.

19. The method according to claim 18, wherein the administration is intravenous administration.

20. A medicament comprising the composition according to any one of claims 1 to 11 for use in the treatment of a CKAP5-associated disease.

21. The medicament for use according to claim 20, wherein the medicament is for intravenous administration.

22. A therapeutic agent for a cancer, the agent comprising the composition according to any one of claims 1 to 11.

23. The therapeutic agent for a cancer according to claim 22, which is for intravenous administration.

[Fig. 1]

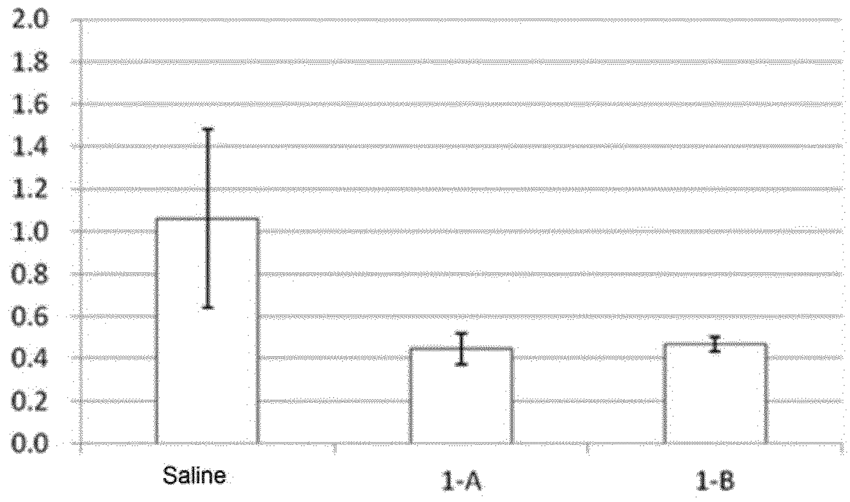
2.0
1.8
1.6
1.4
1.2
1.0
0.8
0.6
0.4
0.2
0.0
Saline
1-A
1-B

[Fig. 2]

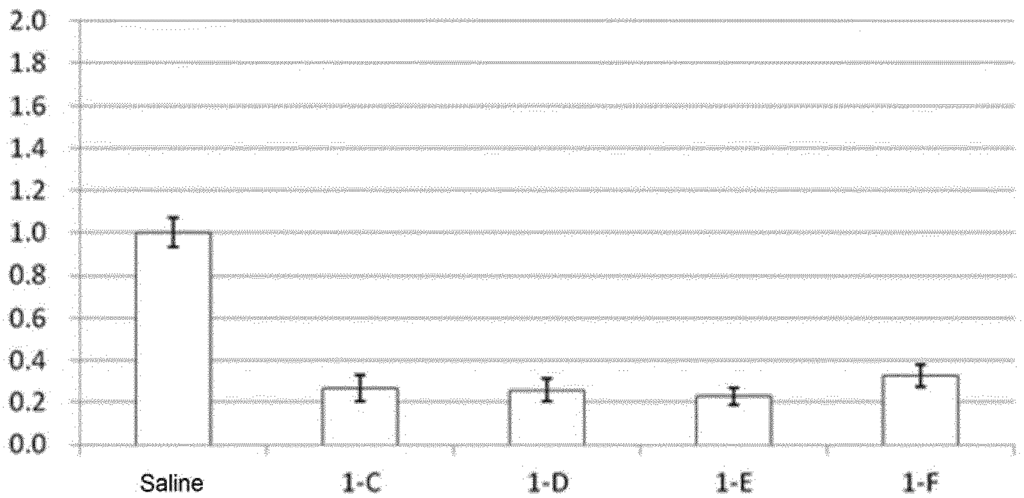
2.0
1.8
1.6
1.4
1.2
1.0
0.8
0.6
0.4
0.2
0.0
Saline
1-C
1-D
1-E
1-F

[Fig. 3]

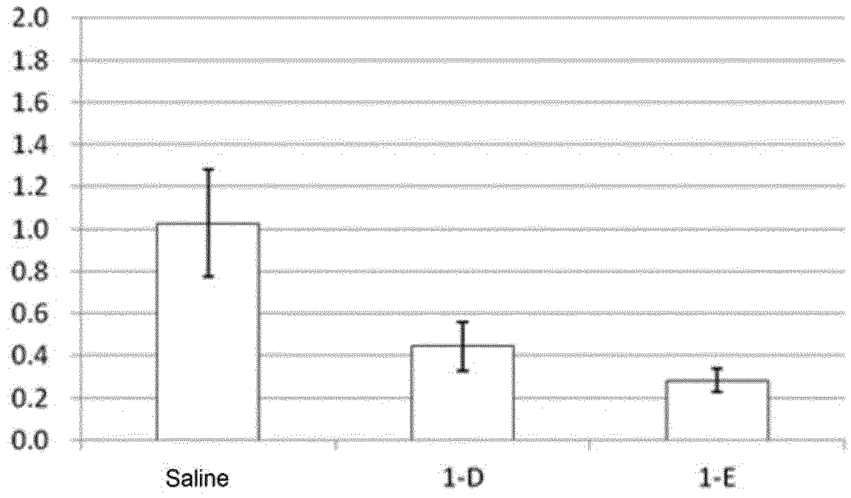
2.0
1.8
1.6
1.4
1.2
1.0
0.8
0.6
0.4
0.2
0.0
Saline
1-D
1-E

[Fig. 4]

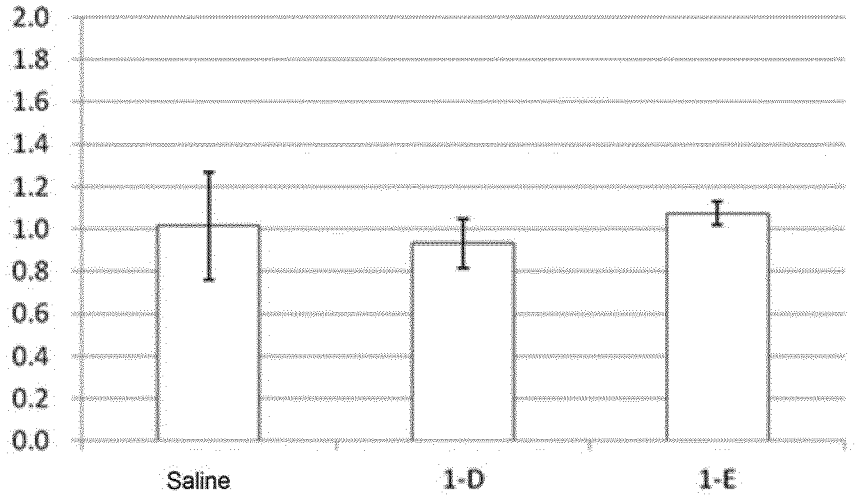
2.0
1.8
1.6
1.4
1.2
1.0
0.8
0.6
0.4
0.2
0.0
Saline
1-D
1-E

[Fig. 5]

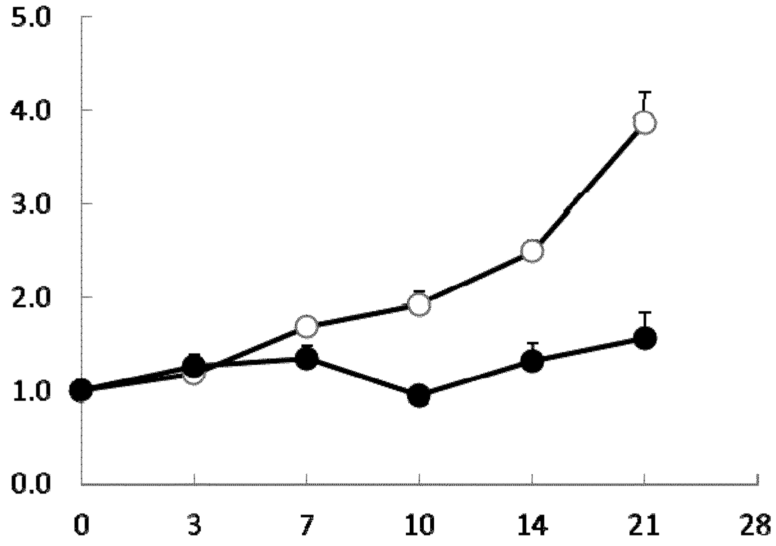


[Fig. 6]

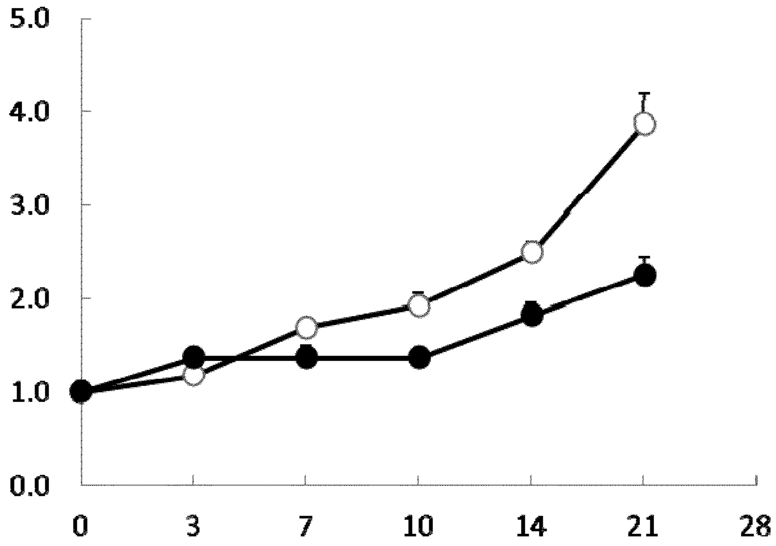

[Fig. 7]

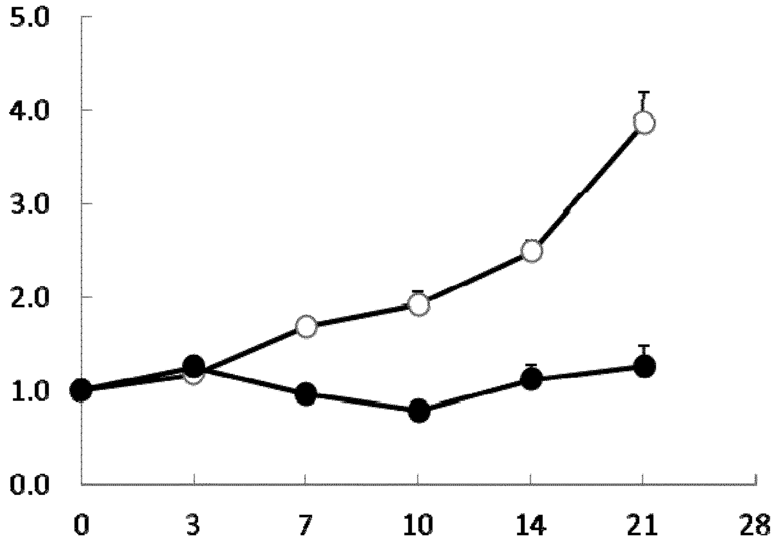


[Fig. 8]

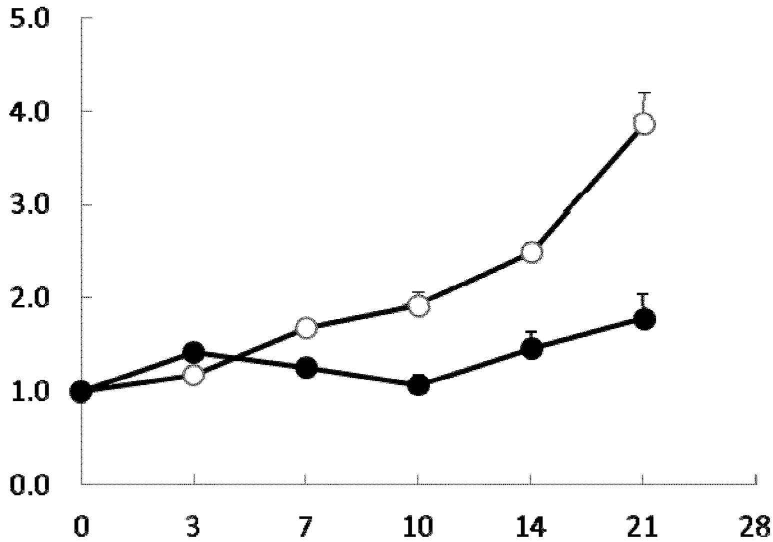

[Fig. 9]

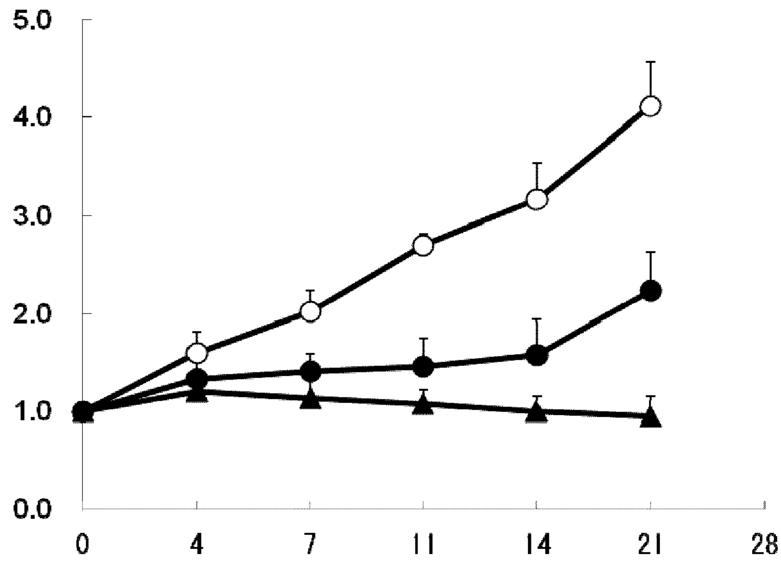


[Fig. 10]

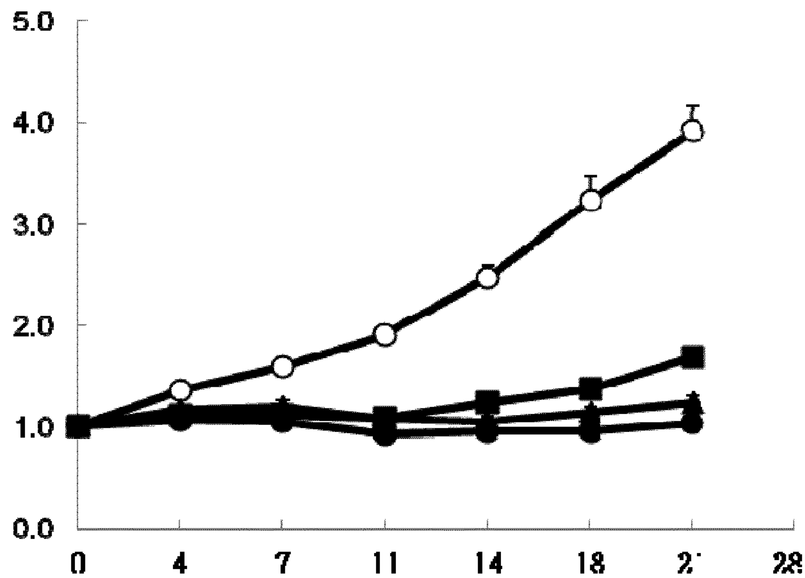

[Fig. 11]

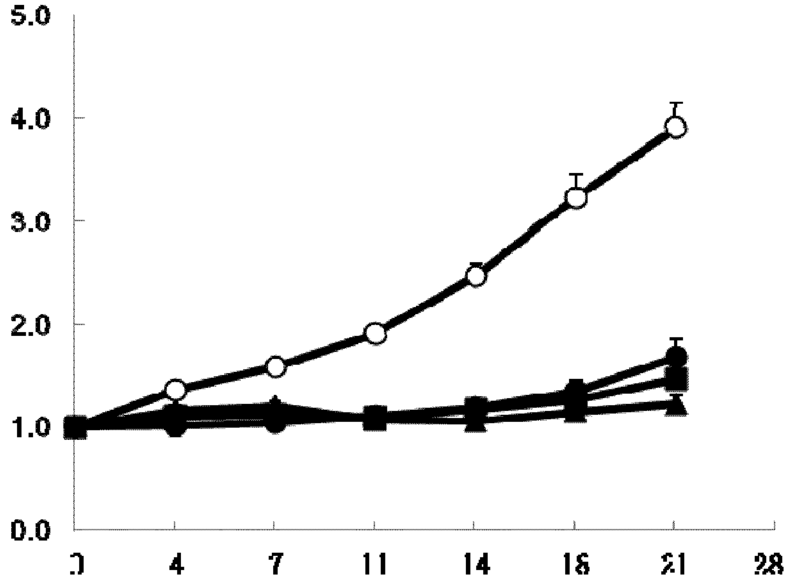

## INTERNATIONAL SEARCH REPORT

International application No.
PCT/JP2015/066134

A. CLASSIFICATION OF SUBJECT MATTER
*A61K31/713*(2006.01)i, *A61K9/127*(2006.01)i, *A61K47/18*(2006.01)i, *A61K48/00*(2006.01)i, *A61P35/00*(2006.01)i

According to International Patent Classification (IPC) or to both national classification and IPC

B. FIELDS SEARCHED

Minimum documentation searched (classification system followed by classification symbols)
A61K31/713, A61K9/127, A61K47/18, A61K48/00, A61P35/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/MEDLINE/EMBASE/BIOSIS(STN), JSTPlus/JMEDPlus/JST7580(JDreamIII), GenBank/EMBL/DDBJ/GeneSeq

C. DOCUMENTS CONSIDERED TO BE RELEVANT

| Category* | Citation of document, with indication, where appropriate, of the relevant passages | Relevant to claim No. |
|---|---|---|
| Y | US 2014/0039032 A1 (Kyowa Hakko Kirin Co., Ltd.),<br>06 February 2014 (06.02.2014),<br>claims; paragraph [0001]; referential example 1; examples 6, 9; test examples 3, 4; fig. 3 to 5<br>& WO 2013/089151 A1 & EP 2792367 A1<br>& TW 1330875 A | 1-23 |
| Y | JP 2006-507841 A (Dharmacon, Inc.),<br>09 March 2006 (09.03.2006),<br>sequence listing<br>& WO 2004/045543 A2 & EP 01560931 A1<br>& US 2005/0108685 A1 & AU 2012333477 A<br>& AT 517992 T & DK 2284266 T<br>& PT 2284266 T & ES 2440284 T<br>& SI 2284266 T | 1-23 |

☒ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
31 August 2015 (31.08.15)

Date of mailing of the international search report
08 September 2015 (08.09.15)

Name and mailing address of the ISA/
Japan Patent Office
3-4-3,Kasumigaseki,Chiyoda-ku,
Tokyo 100-8915,Japan

Authorized officer

Telephone No.

Form PCT/ISA/210 (second sheet) (July 2009)

**INTERNATIONAL SEARCH REPORT**

International application No.
PCT/JP2015/066134

C (Continuation). DOCUMENTS CONSIDERED TO BE RELEVANT

| Category* | Citation of document, with indication, where appropriate, of the relevant passages | Relevant to claim No. |
|---|---|---|
| A | WO 2011/136368 A1 (Kyowa Hakko Kirin Co., Ltd.), 03 November 2011 (03.11.2011), claims; examples 109, 110 & EP 02567951 A1 & US 2013/0245475 A1 & AU 2011245987 A & CN 102884041 A & CA 2800818 A & KR 10-2013-0060210 A | 1-23 |
| P,A | WO 2014/093746 A2 (DICERNA PHARMACEUTICALS, INC.), 19 June 2014 (19.06.2014), fig. 1 & TW 1437368 A & CA 2893824 A | 1-23 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0175164 A
- US 2002132788 A
- WO 0310180 A
- WO 2004045543 A
- WO 2005121348 A
- WO 2009086558 A
- WO 2011136368 A
- US 5804683 A, Usman
- US 5831071 A
- US 5998203 A
- US 6117657 A
- US 6353098 B
- US 6362323 B
- US 6437117 B
- US 6469158 B
- US 6111086 A
- US 6008400 A
- JP S61161246 A
- US 5049386 A
- WO 9116024 A
- WO 97019675 A
- JP 2572554 B
- JP 2659136 B
- JP 2002508765 T
- JP 2002501511 T
- WO 0228367 A
- WO 2006080118 A
- WO 2006100036 A


### Non-patent literature cited in the description


- *DNA Res,* vol. 2, 37-43
- *Eur J Biochem,* vol. 234, 406-13
- *Molecular Biology of the Cell,* vol. 15, 1580-1590
- *Molecular and Cellular Biology,* vol. 28, 7199-7211
- *Oncogene,* vol. 22, 8102-16
- *Biochem J,* vol. 363, 195-200
- *Cancer Res,* vol. 72, 757-68
- *Nature,* 1998, vol. 391 (6669), 806-811
- *Nature Genetics,* 2002, vol. 32 (1), 107-108
- *Nature Biotechnology,* 2002, vol. 20 (10), 1006-1010
- **T. W. GREENE.** Protective Groups in Organic Synthesis. John Wiley & Sons Inc, 1999
- Jikken Kagaku Kouza (The Fifth Series of Experimental Chemistry). Yukikagobutsu no Gosei II. Maruzen, 2005, vol. 14, 351
- **JIKKEN KAGAKU KOUZA (THE FIFTH SERIES OF EXPERIMENTAL CHEMISTRY.** Yukikagobutsu no Gosei I. Maruzen, 2005, vol. 13, 374
- Jikken Kagaku Kouza (The Fifth Series of Experimental Chemistry). Yukikagobutsu no Gosei III. Maruzen, 2005, vol. 15, 1
- Jikken Kagaku Kouza (The Fifth Series of Experimental Chemistry). Yukikagobutsu no Gosei III. Maruzen, 2005, vol. 15, 153
- Jikken Kagaku Kouza (The Fifth Series of Experimental Chemistry). Yukikagobutsu no Gosei V. Maruzen, 2005, vol. 17, 186
- **R. C. LAROCK.** Comprehensive Organic Transformations. Vch Verlagsgesellschaft Mbh, 1999
- *J. Am. Chem. Soc.,* 2002, vol. 124 (10), 2092
- *Acc. Chem. Res.,* 1999, vol. 32, 624
- *J. Am. Chem. Soc.,* 2001, vol. 123, 4653
- *J. Am. Chem. Soc.,* 2000, vol. 122, 6900
- *Nucleic Acid Research,* 2004, vol. 32, e175
- *Nucleic Acids Res.,* 1988, vol. 32, 936-948
- *Nucleic Acids Res.,* 1993, vol. 30, 2435-2443
- *J. Mol. Biol.,* 1965, vol. 13, 238-252
- *J. Cell Biol.,* 1975, vol. 66, 621-634
- *FEBS Lett.,* 1979, vol. 99, 210-214
- *Arch. Biochem. Biophys.,* 1981, vol. 212, 186-194
- *Proc. Natl. Acad. Sci. USA,* 1978, vol. 75, 4194-4198
- *Biochimica et Biophysica Acta,* 2001, vol. 1510, 152-166
- **D. D. LASIC ; F. MARTIN.** Stealth Liposomes. CRC Press Inc, 1995, 93-102
- Emulsion and Nanosuspensions for the Formulation of Poorly Soluble Drugs. Scientific Publishers, 1998, 267-294